# EUROPEAN PATENT APPLICATION

(11) **EP 4 759 353 A1**
(43) Date of publication of application: **17.06.2026**
(21) Application number: 25221018.2
(22) Date of filing: 05.12.2025
(51) Int. Cl.: A61N 1/365, A61N 1/372, A61N 1/368, A61B 5/349, A61B 5/00, A61N 1/375

(54) **METHODS AND SYSTEMS FOR MONITORING AV CONDUCTION USING LEADLESS PACEMAKER**

(30) Priority: 09.12.2024 US 202463729890 P; 04.12.2025 US 202519409581
(71) Applicant: Pacesetter, Inc., Sylmar, CA 91342 (US)
(72) Inventor: Mouchawar, Gabriel A, Sylmar, CA 91342 (US); Fishler, Matthew G, Sylmar, CA 91342 (US)
(74) Representative: Barker Brettell Sweden AB

(57) **Abstract**

System (100) for monitoring atrioventricular (AV) conduction and controlling pacing therapy includes determining atrial events for a series of heartbeats over a detection period using a leadless implantable medical device (LIMD) (102) implanted in or on an atrial chamber of a heart (101). Surrogate AV intervals are determined for at least a portion of the atrial events over the detection period based on a signal indicative of ventricular activity. The surrogate AV intervals are trended over time to generate diagnostic data. The diagnostic data is evaluated against one or more criteria stored in at least one of the LIMD (102) or an external device (109), and in response to the evaluation satisfying the one or more criteria, an action is initiated to modify an operational mode of at least one LIMD (102, 104) or to generate an alert. The action includes changing the operational mode of at least one LIMD (102, 104) or transmitting the alert to the external device (109).

## Description

### FIELD OF THE INVENTION

Embodiments of the present disclosure generally relate to systems for monitoring heart function based on a surrogate of atrioventricular (AV) delay.

### BACKGROUND OF THE INVENTION

One or more leadless implantable devices (LIMD) (e.g., leadless pacemakers) can be implanted within a patient to address one or more different heart conditions. For example, a patient may have one LIMD implanted within or on an atrium to pace the atrium as needed, and in some cases, may have a second LIMD implanted within or on a ventricle to pace the ventricle as needed. When two LIMDs are implanted, they can communicate with each other continually or as needed.

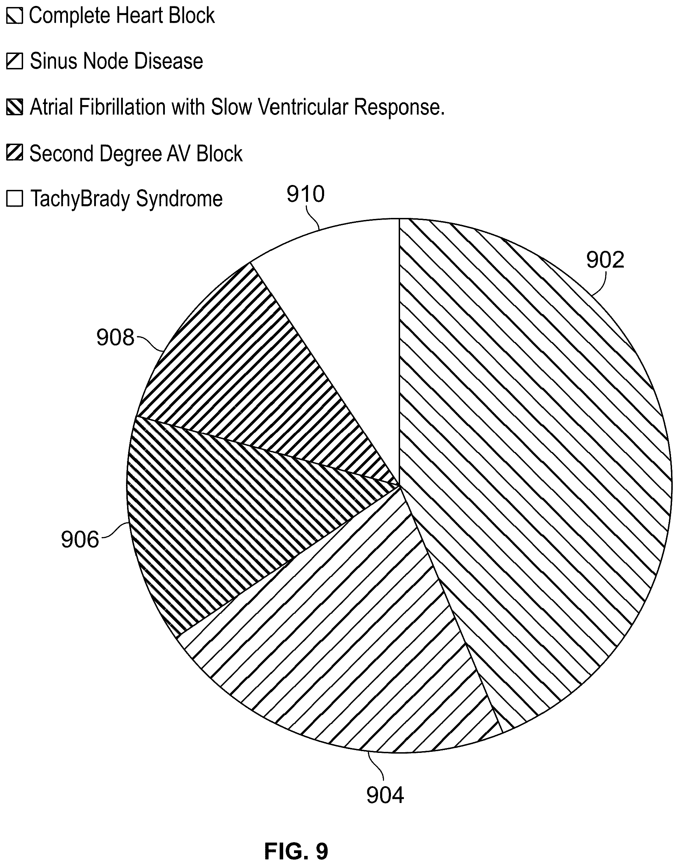

Figure 9 shows a chart displaying an exemplary distribution of patients with cardiac rhythm disease/dysfunction. The cardiac rhythm diseases indicated are complete heart block 802, sinus node disease 804, atrial fibrillation with slow ventricular response 806, second degree atrioventricular (AV) block 808, and tachy/brady syndrome 810. For the approximately 22% of patients experiencing sinus node disease 804 or sick sinus syndrome (SSS), the intrinsic rate of the heart's natural pacemaker (e.g., the sinoatrial (SA) node) can be too slow or irregular, usually with long pauses. It is known from clinical experience that some SSS patients can progress in their conduction disease, such as for example by also developing some level of conduction dysfunction of their AV node. The risk of these SSS patients developing an increasing level of AV block after the initial SSS diagnosis is about five percent per year. Therefore, the cumulative risk is 50 percent over ten years. Consequently, a patient with an initial diagnosis of sinus node disease 804 may thus, over time, ultimately become a patient with complete heart block 802 as well.

Patients having complete heart block 802, as well as patients with any symptomatic level of AV conduction dysfunction, may require two LIMDs, one in an atrium and one in a ventricle, to ensure adequate treatment. Each of the LIMDs can be configured to deliver pacing pulses either automatically or as needed to a cardiac chamber. However, patients experiencing less severe disease may require less treatment. For example, patients having sinus node disease 804 only may not need two LIMDs to treat their current level of disease.

It is undesirable to overtreat the patient by implanting an unnecessary device and it is undesirable to undertreat the patient by not providing the increased level of treatment when it is needed. Thus, a need remains for improvements that enable using one or more LIMDs that are low-cost, less time-consuming, less operator-dependent, and/or potentially non-invasive to evaluate changes to AV delay over time, make timely recommendations for treatment, and/or modify the treatment.

### SUMMARY

In accordance with embodiments herein, a system for monitoring atrioventricular (AV) conduction and controlling pacing therapy is provided. The system comprises a leadless implantable medical device (LIMD) configured to be implanted in or on an atrial chamber of a heart; a memory storing program instructions; and one or more processors that, when executing the program instructions, are configured to utilize the program instructions to: determine atrial events for a series of heartbeats over a detection period using the LIMD; determine surrogate AV intervals for at least a portion of the atrial events over the detection period based on a signal indicative of ventricular activity; trend the surrogate AV intervals over time to generate diagnostic data; evaluate the diagnostic data against one or more criteria stored in at least one of the LIMD or an external device; and in response to the evaluation satisfying the one or more criteria, initiate an action to modify an operational mode of at least one LIMD or to generate an alert, wherein the action comprises at least one of: change the operational mode of at least one LIMD or transmit the alert to the external device.

Optionally, wherein the LIMD further comprises electrodes configured to sense a far-field (FF) cardiac activity signal associated with a ventricular chamber, wherein the FF cardiac activity signal includes the signal indicative of ventricular activity.

Optionally, wherein the LIMD further comprises a heart sound (HS) sensor configured to detect a heart sound signal that comprises an S1 heart sound, wherein the signal indicative of ventricular activity is based on the heart sound signal.

Optionally, wherein the one or more processors are further configured to determine the atrial events over a second detection period; determine the surrogate AV intervals over the second period; and update the trending of the surrogate AV intervals over time to generate updated diagnostic data.

Optionally, wherein the one or more processors are further configured to transmit the diagnostic data or the surrogate AV intervals to the external device.

Optionally, wherein the one or more processors are further configured to: transition the LIMD from a non-dual chamber operating mode to a dual-chamber operating mode; receive the signal indicative of ventricular activity from a second LIMD; and restore a previous operational mode of the LIMD at an expiration of the detection period.

Optionally, the one or more processors are further configured to enable implant-to-implant (i2i) communication between the LIMD and the second LIMD; and in response to restoring the previous operation mode, deactivate the i2i communication.

Optionally, wherein the one or more processors are further configured to generate the alert to prompt at least one of: reprogramming the LIMD, implanting a second LIMD in a ventricular chamber, or reprogramming a second LIMD implanted in a ventricular chamber.

Optionally, wherein the evaluate the diagnostic data comprises applying a machine learning model trained to classify progression of AV conduction disease.

Optionally, wherein the one or more processors are further configured to determine that no surrogate AV interval associated with a first atrial event can be determined when the signal indicative of ventricular activity is not detected within a predetermined timeout window following the first atrial event or when a second atrial event is detected before i) the signal indicative of ventricular activity is detected or ii) the predetermined timeout window expires.

Optionally, wherein the atrial events comprise at least one of atrial pace (AP) events or atrial sense (AS) events.

Optionally, wherein the one or more criteria is programmable.

Optionally, wherein the diagnostic data comprises at least one of i) average deviation AV interval; ii) standard deviation AV interval, iii) minimum and maximum AV interval, iv) average of the difference between AV intervals from consecutive cardiac cycles, v) standard deviation of the difference between AV intervals from consecutive cardiac cycles, vi) percentage of cycles without FF CA detection, vii) binned histograms of AV intervals, or viii) binned histograms of differences between AV intervals from consecutive cardiac cycles.

Optionally, wherein the diagnostic data can be managed as moving-window statistics or exponentially filtered statistics.

Optionally, wherein the electrodes further comprise at least three electrodes, and the one or more processors are further configured to select two of the at least three electrodes to maximize the FF cardiac activity signal relative to the atrial events.

### BRIEF DESCRIPTION OF THE DRAWINGS

Figure 1A illustrates a system including one or more leadless implantable medical device (LIMD) implanted in a heart in accordance with embodiments herein.
Figure 1B is a block diagram showing exemplary electronics within the LIMD in accordance with embodiments herein.
Figure 1C illustrates a side view of the LIMD in accordance with embodiments herein.
Figure 2A shows an LIMD including a hermetic housing with electrodes disposed thereon in accordance with embodiments herein.
Figure 2B illustrates an LIMD having three electrodes in accordance with embodiments herein.
Figure 3A and 3B illustrate computer-implemented and/or processor-implemented methods that monitor AV conduction in a patient and control pacing therapy of the patient.
Figure 4 illustrates another computer-implemented and/or processor-implemented method that, using an electrocardiogram (ECG), monitors AV conduction in a patient and controls pacing therapy of the patient.
Figure 5 illustrates a graph of an ECG and associated marker channels as acquired from an LIMD by an external device in accordance with embodiments herein.
Figure 6 illustrates a computer-implemented and/or processor-implemented method that monitors AV conduction in a patient and controls pacing therapy of the patient having two LIMDs implanted.
Figure 7 illustrates a computer-implemented and/or processor-implemented method that, using an LIMD and an implanted HS sensor, monitors AV conduction in a patient and controls pacing therapy of the patient.
Figure 8 illustrates still another computer-implemented and/or processor-implemented method that monitors AV conduction in a patient and controls pacing therapy of the patient.
Figure 9 shows a chart displaying different diagnoses of sick sinus syndrome (SSS) patients who have at least some degree of heart block or increase in AV delay.

### DETAILED DESCRIPTION

It will be readily understood that the components of the embodiments as generally described and illustrated in the Figures herein, may be arranged and designed in a wide variety of different configurations in addition to the described example embodiments. Thus, the following more detailed description of the example embodiments, as represented in the Figures, is not intended to limit the scope of the embodiments, as claimed, but is merely representative of example embodiments.

Reference throughout this specification to "one embodiment" or "an embodiment" (or the like) means that a particular feature, structure, or characteristic described in connection with the embodiment is included in at least one embodiment. Thus, appearances of the phrases "in one embodiment" or "in an embodiment" or the like in various places throughout this specification are not necessarily all referring to the same embodiment.

Furthermore, the described features, structures, or characteristics may be combined in any suitable manner in one or more embodiments. In the following description, numerous specific details are provided to give a thorough understanding of embodiments. One skilled in the relevant art will recognize, however, that the various embodiments can be practiced without one or more of the specific details, or with other methods, components, materials, etc. In other instances, well-known structures, materials, or operations are not shown or described in detail to avoid obfuscation. The following description is intended only by way of example, and simply illustrates certain example embodiments.

The methods described herein may employ structures or aspects of various embodiments (e.g., systems, devices and/or methods) discussed herein. In various embodiments, certain operations may be omitted or added, certain operations may be combined, certain operations may be performed simultaneously, certain operations may be performed concurrently, certain operations may be split into multiple operations, certain operations may be performed in a different order, or certain operations or series of operations may be re-performed in an iterative fashion. It should be noted that other methods may be used, in accordance with an embodiment herein. Further, wherein indicated, the methods may be fully or partially implemented by one or more processors of one or more devices or systems. While the operations of some methods may be described as performed by the processor(s) of one device, additionally, some or all such operations may be performed by the processor(s) of another device described herein. For example, an implantable medical device (IMD) includes IMD memory and one or more IMD processors, while each external device/system (ED) (e.g., local, remote, or anywhere within the healthcare system) includes ED memory and one or more ED processors.

### Terms

The term "Sick sinus syndrome" or "SSS", also known as sinus node dysfunction, shall mean a disorder of the sinoatrial node caused by impaired SA node function and impulse transmission producing a constellation of abnormal rhythms. These include atrial brady-arrhythmias, atrial tachyarrhythmias and, sometimes, bradycardia alternating with tachycardia often referred to as tachy-brady syndrome. These arrhythmias may result in palpitations and decreased tissue perfusion and consequent fatigue, lightheadedness, pre-syncope, and syncope.

The terms "AV interval" and "surrogate AV interval", shall mean a time interval between an atrial event associated with activation of an atrium (e.g., right atrium) and a characteristic of interest (COI) of a ventricular event associated with activation of a ventricle (e.g., R-wave, FF R-wave, peak or start of R-wave, heart sound such as S1, ventricular sense (VS), ventricular pulse (VP)) is detected/determined. The time interval is a surrogate for intrinsic AV delay and in some cases may be the same as the intrinsic AV delay. The atrial and ventricular events can be sensed or paced events. Different surrogate AV intervals may be measured depending upon the selected atrial and ventricular events (e.g., sensed and/or paced events, different COIs).

The terms "AV conduction disease" and "heart block" shall mean a slowing or lack of the electrical signals that move from the atrium to the ventricle. First-degree heart block indicates a relatively longer delay than normal of the electrical signals as the electrical signals move from the atrium to the ventricle and may not require treatment. Second-degree heart block involves intermittent failure of AV conduction. Type I is characterized by progressively increasing PR intervals over multiple cardiac cycles until an atrial activation fails to be conducted to the ventricles during one cardiac cycle. Type II is characterized by relatively constant (normal or prolonged) PR interval of all conducted atrial activations, followed by sudden failure of a P wave to be conducted to the ventricles. Third-degree heart block means that the electrical signals do not conduct from the atria to the ventricles, resulting in no pulse or a very slow pulse. See https://www.hopkinsmedicine.org/health/conditions-and-diseases/heart-block. In some cases, first-degree heart block can be diagnosed when the p-wave to R-wave interval (PR interval) is greater than approximately 200 milliseconds (ms).

The term "posture" refers to postural states of a patient including supine, laying on a right side, laying on a left side, sitting, standing, and the like.

The term "activity level" refers to intensity and/or types of activity currently experienced by a patient at a point in time, including stationary state, rest state, exercise state, walking state, and the like.

The terms "cardiac activity signal", "cardiac activity signals", "CA signal" and "CA signals" (collectively "CA signals") are used interchangeably throughout to refer to an analog or digital electrical signal recorded by two or more electrodes positioned on, near, or in the heart, subcutaneous, or cutaneous, where the electrical signals are indicative of cardiac electrical activity. The cardiac activity may be normal/healthy or abnormal/arrhythmic. Non-limiting examples of CA signals include ECG signals collected by cutaneous and/or subcutaneous electrodes, and intracardiac electrogram (IEGM) signals collected by electrodes positioned within or proximate to the heart wall and/or chambers of the heart.

The term seismocardiography (SCG) refers to a recording of vibrations induced by a heartbeat. SCG signals can be detected and recorded by a heart sound sensor, such as an accelerometer, and can generally be referred to as heart sound (HS) signals.

The terms "health care system" and "digital health care system" are used interchangeably throughout to refer to a system that includes equipment for measuring health parameters, and communication pathways from the equipment to secondary devices. The secondary devices may be at the same location as the equipment, or remote from the equipment at a different location. The communication pathways may be wired, wireless, over the air, cellular, in the cloud, etc. In one example, the healthcare system provided may be one of the systems described in U.S. Pat. Pub. No. 2021/0020294 titled METHODS DEVICE AND SYSTEMS FOR HOLISTIC INTEGRATED HEALTHCARE PATIENT MANAGEMENT. Other patents that describe example monitoring systems include U.S. Pat. No. 6,572,557; titled SYSTEM AND METHOD FOR MONITORING PROGRESSION OF CARDIAC DISEASE STATE USING PHYSIOLOGIC SENSORS; U.S. Pat. No. 6,480,733 titled METHOD FOR MONITORING HEART FAILURE filed Dec. 17, 1999, to Turcott; U.S. Pat. No. 7,272,443 titled SYSTEM AND METHOD FOR PREDICTING A HEART CONDITION BASED ON IMPEDANCE VALUES USING AN IMPLANTABLE MEDICAL DEVICE, filed Dec. 14, 2004, to Min et al; U.S. Pat. No. 7,308,309 titled DIAGNOSING CARDIAC HEALTH UTILIZING PARAMETER TREND ANALYSIS, filed Jan. 11, 2005, to Koh; and U.S. Pat. No. 6,645,153 titled SYSTEM AND METHOD FOR EVALUATING RISK OF MORTALITY DUE TO CONGESTIVE HEART FAILURE USING PHYSIOLOGIC SENSORS, filed Feb. 7, 2002, to Kroll et. al.

The terms "artificial intelligence", "AI", "machine learning", and "convolutional neural networks" are used interchangeably throughout and shall mean an artificial intelligence algorithm that learns from various automatic or manual inputs, such as features of interest (e.g., COIs, sensed or paced events), prior device classified degrees of AV delay, surrogate AV intervals, and/or heart block, observations and/or data. By way of example, a machine learning algorithm can be trained to classify progression of AV conduction disease. The machine learning algorithm is adjusted over multiple iterations based on the features or characteristics of interest, posture, heart sound signals, S1 COM, S2 COM, EMAT, SI, CA signals, characteristics of interest of the CA signals, prior device classified arrhythmias, observations and/or data. For example, the machine learning algorithm is adjusted by supervised learning, unsupervised learning, and/or reinforcement learning. Non-limiting examples of machine learning algorithms are a convolutional neural network, gradient boosting random forest, decision tree, K-means, deep learning, artificial neural network, and/or the like.

The terms "RA", "LA", "RV", and "LV" shall mean the right atrium, left atrium, right ventricle, and left ventricle, respectively.

The abbreviations "AP" and "VP" refer to an atrium paced event and a ventricle paced event, respectively.

The abbreviations "AS" and "VS" refer to an atrium sensed event and a ventricle sensed event, respectively.

The term "operational mode" refers to the mode of the LIMD. When referring to various types of operational modes or pacing schemes, three letters are often used to refer to the type of pacing. In other words, a three position pacemaker code is often used, with the following nomenclature followed: the first position refers to the cardiac chamber paced; the second position refers to the cardiac chamber sensed; and the third position refers to the response to a sensed event. In the first and second positions, the letter O means none, the letter A means Atrium, the letter V means Ventricle, and the letter D means Dual (i.e., A and V). In the third position the letter O means none, the letter I means Inhibited, the letter T means Triggered (aka Tracked), and the letter D means Dual (i.e., T+I). For example, DDD pacing uses two LIMDs, wherein, in some embodiments, an LIMD is implanted in (or on) a patient's RV chamber and is used to perform VDD pacing, and another LIMD is implanted in (or on) the patient's RA chamber and is used to perform AAI, or ADI pacing. DDD pacing is a dual chamber pacing mode and relies on synchronization between the LIMDs, referred to as implant-to-implant ("i2i") communication. Non-dual pacing modes such as AAI, VVI, AAI+VVI, etc., do not require i2i communication.

The term "leadless" generally refers to an absence of electrically conductive leads that traverse vessels or other anatomy outside of the intra-cardiac space, while "intra-cardiac" means generally, entirely within the heart and associated vessels, such as the superior vena cava (SVC), inferior vena cava (IVC), coronary sinus (CS), coronary veins (CV), pulmonary arteries, and the like.

The term "COI" refers to a characteristic of interest within a signal. Non-limiting examples of a COI from a CA signal with a PQRST complex, include an R-wave, P-wave, T-wave, and isoelectric segments. Non-limiting examples of a COI from CA signals collected at an individual electrode(s) include a sensed event (e.g., an intrinsic event or evoked response) and/or paced event. The COI may correspond to a peak of an individual sensed event, R-wave, beginning of R-wave, an average or median P, R or T-wave peak, and the like. In another example, the COI may include COIs of heart sound (HS) signals including S1 amplitudes, S2 amplitudes, S3 amplitudes, S4 amplitudes, S1 timing such as start of S1, peak of S1, etc., S1 to S2 intervals, S2 to S3 intervals, S3 to S4 intervals, S4 to S1 intervals, or the like.

The terms "processor" and "one or more processors" shall mean one or more processors. The one or more processors may be implemented by one, or by a combination of more than one implantable medical device, a wearable device, a local device, a remote device, a server computing device, a network of server computing devices and the like. The one or more processors may be implemented at a common location or at distributed locations. The one or more processors may implement the various operations described herein in a serial or parallel manner, in a shared-resource configuration and the like.

### Overview

In accordance with new and unique aspects herein, methods, devices, and systems are described that utilize signals acquired by one or more leadless pacemakers (LIMD) and in some embodiments a heart sound (HS) sensor, such as an accelerometer, or ECG, to monitor atrioventricular (AV) delay and determine whether the patient is experiencing a progression of AV conduction disease or an increase in surrogate AV intervals that requires treatment or a change of treatment. The HS sensor can be, for example, a miniaturized accelerometer using micro-electro-mechanical-system (MEMS) technology that can be included within an LIMD. In some cases, the signals acquired by the accelerometer can be referred to as seismocardiography (SCG) signals.

An advantage is realized as a single LIMD can be implanted within an atrial chamber and used to sense both near-field (NF) and far-field (FF) cardiac activity (CA) signals. The NF CA signals are associated with the local chamber of the heart (e.g., atrial chamber) while the FF CA signals are associated with a remote chamber of the heart (e.g., ventricle). The LIMD can identify atrial events, such as atrial pace (AP) and atrial sense (AS) events, and a characteristic of interest (COI) associated with the FF CA signals, such as related to the R-wave. The LIMD can determine surrogate AV intervals (e.g., time intervals) between the atrial events and COIs over a time period. The surrogate AV intervals are trended over time to generate diagnostic data that is evaluated against one or more criteria. If one or more criteria are satisfied, an action is initiated, such as modifying an operational mode of an LIMD or generating an alert. The alert may indicate a need for additional monitoring and/or treatment, such as an indication that a ventricular LIMD should be implanted, etc. An alert can also transmit or prepare for transmission upon connection with an external device, associated trended and/or summary diagnostic data that can be displayed on an external device.

In some cases, the LIMD will not reliably detect the R-wave and can utilize the HS sensor to detect HS signals. The COI can be, for example, the S1 signal. The LIMD can similarly determine the surrogate AV intervals, generate alerts, trend data acquired over time, etc.

Some patients have an LIMD implanted within the atrial chamber and a second LIMD implanted within a ventricular chamber that are not programmed to continuously communicate with each other. In this example, the atrial LIMD can periodically and temporarily automatically transition to a dual chamber mode (e.g., DDD, etc.), initiating bidirectional implant-to-implant (i2i) communication. The atrial LP collects the timings of the ventricular pace (VP) and/or ventricular sense (VS) events via the ongoing i2i communication and determines the associated surrogate AV intervals. When the measurements are complete, the LIMDs can automatically transition back to their previous operational mode.

In other cases, one of the LIMDs can determine, based on the surrogate AV intervals, that the patient may benefit from an increased level of treatment. The LIMDs may automatically retain their operational modes (e.g., DDD) and continue i2i communication.

The LIMD(s) can communicate with an external device, such as a smartphone, programmer, etc., which in some cases can receive determined and/or acquired signal data and process some or all of the data. In some other cases, the data may not be processed by the external device, but instead be conveyed over the internet, etc., to a central processing system or other processing system for data processing, storage, etc. The external device can convey data over the internet (wired and/or wirelessly), local area networks, etc., to transmit alerts, trended surrogate AV intervals (e.g., diagnostic data), treatment and/or monitoring recommendations, and the like to clinicians, data storage, AI processors, etc.

In accordance with new and unique aspects herein, a technical advantage of identifying AV delay dysfunction is provided. The CA and HS signals and paced events can be acquired with little or no action required of the patient, and can be acquired anywhere outside or inside of a medical facility without the assistance of medical personnel. The patient can receive timely intervention and treatment as alerts are generated when surrogate AV interval measurements have satisfied criteria that indicates a progression of the degree of AV conduction disease. Operational modes and settings of the LIMD(s) can be automatically programmed and/or treatments can be deployed (e.g., pacing, medication) based on the alerts. Treatments can also be adjusted/implemented remotely without action by the patient or clinician.

### Leadless Implantable Medical Device

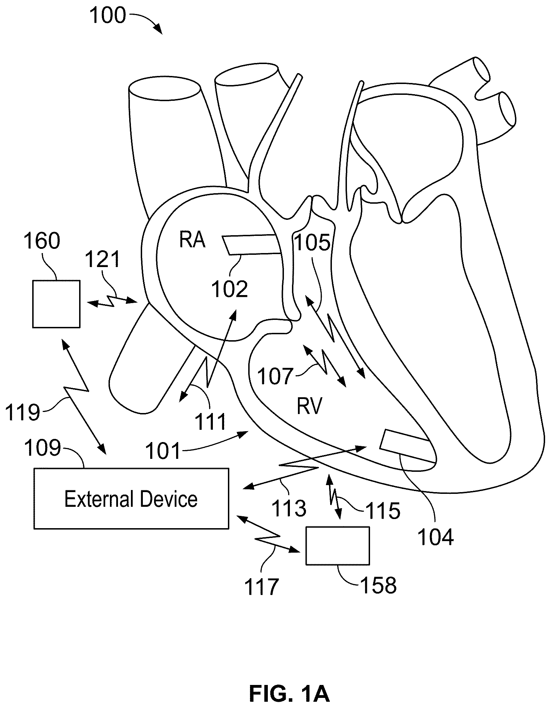

Figure 1A illustrates a system 100 formed in accordance with embodiments herein as implanted in a heart 101. The system 100 comprises one or more leadless implantable medical devices (LIMD), such as LIMD 102 shown implanted in or on the right atrial (RA) chamber (e.g., right atrium) and LIMD 104 shown implanted in or on the right ventricular (RV) chamber (e.g., right ventricle). Optionally, additional LIMDs may be implanted in the left atrium and/or the left ventricle. Alternatively, the LIMDs 102, 104 may be implanted in other combinations of chambers, such as one LIMD in the right atrium and a second LIMD in the left atrium, the left atrium and left ventricle, respectively, or in the right ventricle and left ventricle, respectively, and the like.

If more than one LIMD is implanted, the LIMDs 102 and 104 can communicate with one another to inform one another of various local physiologic activities, such as local intrinsic events, local paced events and the like. LIMDs 102 and 104 may be constructed in a similar manner, but operate differently, e.g., can be programmed with a different operational mode, based upon which chamber LIMD 102 or 104 is located in and the desired functionality. It is noted that the terms "cardiac chamber" and "chamber of the heart" are used interchangeably herein.

In some embodiments, if two LIMDs are implanted, the LIMD 102 is used to perform dual-chamber pacing, the LIMD 104 is used to perform VDD pacing, and the LIMDs 102 and 104 are collectively used to perform DDD pacing. The dual chamber pacing (performed by the LIMD 102) involves atrial pacing, ventricular and atrial (i.e., dual) sensing, and dual (i.e., triggered and inhibited) response to a sensed event. The VDD pacing (performed by the LIMD 104) involves ventricular pacing, atrial and ventricular (i.e., dual) sensing, and dual (i.e., triggered and inhibited) response to a sensed event. The DDD pacing (performed collectively by the LIMDs 102 and 104) involves atrial and ventricular (i.e., dual) pacing, atrial and ventricular (i.e., dual) sensing, and dual (i.e., triggered and inhibited) response to a sensed event.

Each LIMD 102, 104 uses two or more electrodes located within, on, or within a few centimeters of the housing of the LIMD 102, 104, for pacing and sensing at the cardiac chamber, for bidirectional communication with one another, and for bidirectional communication with an external device 109 (e.g., commercial wireless device, a tablet computer, a smartphone, a laptop computer, smart watch, specialized wireless device, programmer, bedside monitor). In one example, the external device 109 is a proximate external device located proximate to or adjacent to a patient. An external device located in the same room as the patient is a proximate external device. Alternatively, the external device can be a remote external device where the external device is located a long distance from the patient such as at least one-hundred meters, in a different room, in a different building, at least five miles away, at least ten miles away, at least one-hundred miles away, etc.

In some embodiments, the external device 109 can include a heart sound (HS) sensor, such as an accelerometer for acquiring heart sound signals. The external device 109 can be an external cardiac monitor such as a Holter monitor. In some cases, external leads (not shown) can be used to acquire ECG signals. The external device 109 can be affixed to a patient's torso, such as with adhesive, be integrated with and/or held in place with a garment, and/or held in place by the patient or clinician.

In some embodiments, LIMDs 102 and 104 communicate with one another and with the external device 109 through wireless transceivers, communication coils and antenna, and/or by conductive communication through the same electrodes as (or one or more different electrodes than) used for sensing and/or delivery of pacing therapy. When conductive communication is performed using electrodes, the system 100 may omit an antenna or telemetry coil in one or more of LIMDs 102 and 104. When an antenna, wireless transceiver, etc., is used, wireless communication with the external device 109 can be accomplished with one or more predetermined wireless protocols (e.g., Bluetooth^{®}, Bluetooth^{®} low energy, Wi-Fi, etc.). The external device 109 includes at least one of applicable transceiver, antenna, electrodes that can be applied to the patient's skin for conductive communication, and the like configured to communicate bidirectionally with the LIMDs 102, 104.

In Figure 1A, the two LIMDs 102 and 104 are shown as being implanted endocardially, i.e., within respective cardiac chambers. In other words, in Figure 1A each of the LIMDs 102 and 104 is shown as being implanted in a respective cardiac chamber, i.e., the LIMD 102 is shown as being implanted in the RA chamber, and the LIMD 104 is shown as being implanted in the RV chamber. Alternatively, one or both of the LIMDs 102 and 104 can be implanted epicardially (on the external heart surface) by affixing to the exterior surface of the heart. For example, it would also be possible for the LIMD 102 to be affixed to an exterior surface of the RA chamber, in which case the LIMD 102 can be said to be implanted on (rather than in) the RA chamber. Similarly, it would also be possible for the LIMD 104 to be affixed to an exterior of the RV chamber, in which case the LIMD 104 can be said to be implanted on (rather than in) the RV chamber. More generally, an LIMD can either be implanted in or on the cardiac chamber that the LIMD is being used to pace. It is noted that the terms "implanted in," "implanted within," "located in," and "located within" are used interchangeably herein when referring to where a particular LIMD is implanted. Further, it is noted that the terms "located on" and "implanted on" are used interchangeably herein when referring to where a particular LIMD is implanted. The cardiac chamber within or on which a particular LIMD is implanted can be referred to as a "local chamber", while another chamber (within or on which the particular LIMD is not implanted) can be referred to as a "remote chamber".

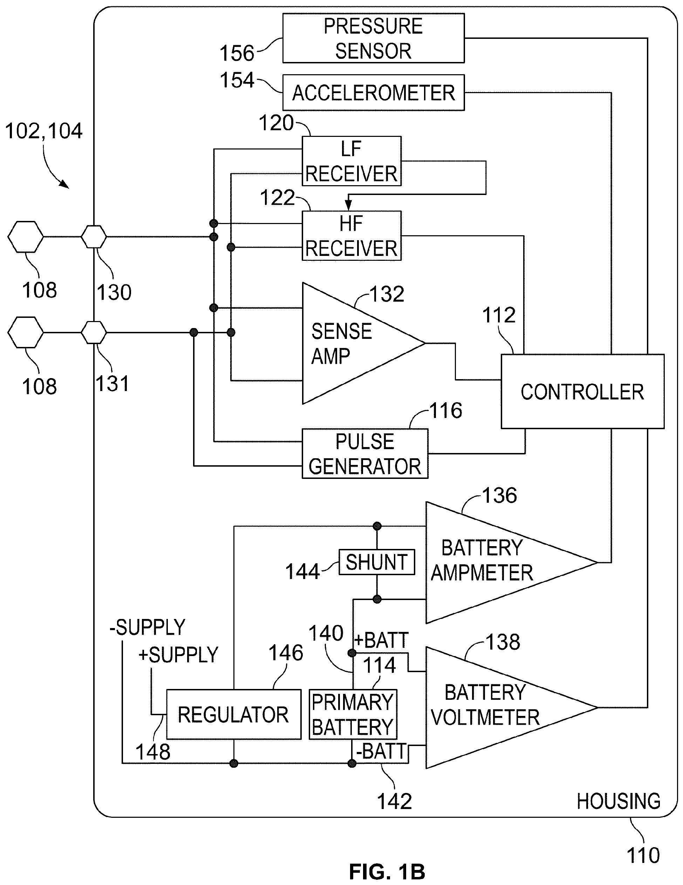

Referring to Figure 1B, a block diagram shows exemplary electronics within LIMDs 102, 104 in accordance with embodiments herein. LIMD 102, 104 includes first and second receivers 120 and 122 that collectively define separate first and second communication channels 105 and 107 (Figure 1A), (among other things) between LIMDs 102 and 104. Although first and second receivers 120 and 122 are depicted, in other embodiments, LIMD 102, 104 may only include first receiver 120, or may include additional receivers other than first and second receivers 120 and 122. As will be described in additional detail below, the pulse generator 116 can function as a transmitter that transmits implant-to-implant (i2i) communication signals using the electrodes 108. Usage of the electrodes 108 for communication enables the one or more LIMDs 102, 104 to perform antenna-less and telemetry coil-less communication.

In some embodiments, when both of the LIMDs 102, 104 are implanted and i2i communication is enabled, when one of the LIMDs 102, 104 senses an intrinsic event or delivers a paced event, the corresponding LIMD 102, 104 transmits an implant event message to the other LIMD 102, 104. For example, when an atrial LIMD 102 senses/paces an atrial event, the atrial LIMD 102 transmits an implant event message including an event marker indicative of a nature of the event (e.g., intrinsic/sensed atrial event, paced atrial event). When a ventricular LIMD 104 senses/paces a ventricular event, the ventricular LIMD 104 transmits an implant event message including an event marker indicative of a nature of the event (e.g., intrinsic/sensed ventricular event, paced ventricular event). In certain embodiments, LIMD 102, 104 transmits an implant event message to the other LIMD 102, 104 preceding the actual pace pulse so that the remote LIMD can blank its sense inputs in anticipation of that remote pace pulse (to prevent inappropriate crosstalk sensing).

Still referring to Figure 1B, each LIMD 102, 104 is shown as including a controller 112 and a pulse generator 116. The controller 112 can include, e.g., a microprocessor (or equivalent control circuitry), RAM and/or ROM memory, logic and timing circuitry, state machine circuitry, and I/O circuitry, but is not limited thereto. The controller 112 can further include, e.g., timing control circuitry to control the timing of the stimulation pulses (e.g., pacing rate, atrio-ventricular (AV) delay, atrial interconduction (A-A) delay, or ventricular interconduction (V-V) delay, etc.). Such timing control circuitry may also be used for the timing of refractory periods, blanking intervals, noise detection windows, evoked response windows, alert intervals, marker channel timing, and so on. The controller 112 can further include other dedicated circuitry and/or firmware/software components that assist in monitoring various conditions of the patient's heart and managing pacing therapies. The controller 112 and the pulse generator 116 may be configured to transmit event messages, via the electrodes 108, in a manner that does not inadvertently capture the heart in the chamber where LIMD 102, 104 is located, such as when the associated chamber is not in a refractory state. In addition, an LIMD 102, 104 that receives an event message may enter an "event refractory" state (or event blanking state) following receipt of the event message. The event refractory/blanking state may be set to extend for a determined period of time after receipt of an event message in order to avoid the receiving LIMD 102, 104 from inadvertently sensing another signal as an event message that might otherwise cause retriggering. For example, the receiving LIMD 102, 104 may detect a measurement pulse from another LIMD 102, 104 or external device 109.

In accordance with certain embodiments herein, referring also to Figure 1A, the external device 109 may communicate over a programmer-to-LIMD channel, with LIMD 102, 104 utilizing the same communication scheme. The external device 109 may listen to the event message transmitted between LIMD 102, 104 and synchronize programmer to implant communication such that external device 109 does not transmit communication signals 111, 113 until after an i2i messaging sequence is completed. Alternatively, the external device 109 may wait for a directed communication message transmitted to the external device 109 from LIMD 102 or 104 that indicates to the external device 109 that that the LIMD is ready to trade communication signals 111, 113 with the external device 109. An LIMD 102, 104 can also communicate with other types of devices, such as, but not limited to, an external monitor 158 (via communication signals 115) such as a patch that may be in communication with the patient's skin and/or be integrated into or secured by a garment, such as a compression shirt, belt, sash, etc., that serves to hold components of the monitor in contact with the patient's torso. The external monitor 158 can also communicate via communication signals 117 with the external device 109. Other devices such as a medication delivery device 160 is also shown and can communicate with the external device 109 (via communication signals 119) and/or the LIMDs 102, 104 (via communication signals 121). The medication delivery device 160 can be implantable or affixed to the skin of the patient and is capable of dynamically delivering a controlled amount of medicine. In some embodiments, based on the analysis of the signals detected by the LIMDs 102, the external device 109 and/or LIMDs 102, 104 can direct the medication delivery device 160 to increase, decrease, start, and/or stop delivering one or more medicines, such as Atropine, hormones, Anticholinergic agents, Beta1/Beta2 adrenergic agonists, Catecholiamines, etc.

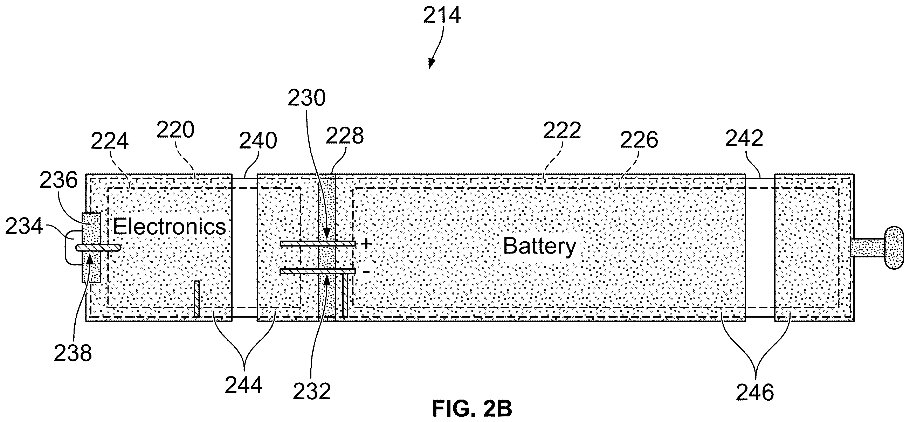

In some embodiments, the individual LIMD 102, 104 can comprise a hermetic housing 110 configured for placement on or attachment to the inside or outside of a cardiac chamber and at least two electrodes 108 proximal to the housing 110 and configured for bidirectional communication with at least one other device 109 outside the body, a device in communication with the skin of the body, or a device within the body, such as another implantable medical device. As will be described in additional detail below, with reference to Figure 2B, in certain embodiments an individual LIMD includes two hermetic housings, one of which includes electronic circuitry, and the other of which includes a battery.

Referring to Figure 1B, the LIMD 102, 104 is shown as including a heart sound (HS) sensor, such as an accelerometer 154 and/or a pressure sensor 156, which can be hermetically contained within the housing 110. The accelerometer 154 can be any one of various different types of well-known accelerometers, or can be a future developed accelerometer. For one example, the accelerometer 154 can be or include, e.g., a MEMS (micro-electromechanical system) multi-axis accelerometer of the type exploiting capacitive or optical cantilever beam techniques, or a piezoelectric accelerometer that employs the piezoelectric effect of certain materials to measure dynamic changes in mechanical variables. Where the accelerometer is a multi-axis accelerometer it can include two or three sensors aligned along orthogonal axes. Exemplary multi-axis accelerometers (also referred to as multi-dimensional accelerometers) that can be used are described in U.S. Pat. No. 6,658,292 (Kroll et al.) and U.S. Pat. No. 6,466,821 (Pianca et al.). For another example, a commercially available micro-electromechanical system (MEMS) accelerometer marketed as the ADXL345 by Analog Devices, Inc. (headquartered in Norwood, Mass.) is a three-axis accelerometer and includes polysilicon springs that provide a resistance against acceleration forces. The term MEMS has been defined generally as a system or device having micro-circuitry on a tiny silicon chip into which some mechanical device such as a mirror or a sensor has been manufactured. The aforementioned ADXL345 includes a micromachined accelerometer co-packaged with a signal processing IC. In another embodiment, the accelerometer is formed and operates in the manner described in US patent 6,937,900, titled AC/DC MULTI-AXIS ACCELEROMETER FOR DETERMINING A PATIENT ACTIVITY AND BODY POSITION, and US Patent Publication 2021/0350931, titled METHOD AND SYSTEM FOR HEART CONDITION DETECTION USING AN ACCELEROMETER.

Another commercially available MEMS accelerometer is the ADXL327 by Analog Devices, Inc., which is a small, thin, low power, complete three axis accelerometer with signal conditioned voltage outputs. In the ADXL327, the mechanical sensor and signal conditioning IC are packaged together. A further commercially available MEMS accelerometer that can be used is the LIS3DH three-axis accelerometer by STMicroelectronics (headquartered in Geneva, Switzerland). Additional and/or alternative types of accelerometers may also be used. For example, it is also within the scope of the present technology for the accelerometer 154 to be a beam-type of accelerometer, an example of which is described in U.S. Pat. No. 6,252,335 (Nilsson et al.).

The HS sensor (e.g., accelerometer 154) can be, e.g., a one-dimensional (1D) accelerometer (also known as a one-axis accelerometer), a two-dimensional (2D) accelerometer (also known as a two-axis accelerometer), or a three-dimensional (3D) accelerometer (also known as a three-axis accelerometer). A 1D accelerometer measures acceleration along one axis, e.g., the z-axis. A 2D accelerometer measures acceleration along two axes that are orthogonal to one another, e.g., the z-axis, and the x- or y-axis. A 3D accelerometer measures acceleration along three axes that are orthogonal to one another, e.g., the z-axis, the x-axis, and the y-axis. Each measure of acceleration (i.e., rate of change of velocity) can actually be a measure of proper acceleration, which is the rate of change of velocity of a body in its own instantaneous rest frame.

Where an LIMD 102, 104 includes an accelerometer 154 within a housing 110 of the LIMD 102, 104 or attached thereto, the accelerometer 154 can be used to detect SCG signals which include heart sound signals from which individual heart sounds such as S1, S2, etc., can be determined, as well as used to measure the acceleration of the LIMD 102, 104 along one or more axes, which measurement(s) can be used to determine the orientation of the LIMD 102, 104. Accordingly, because the output(s) of the accelerometer 158 can be used to determine the orientation of the LIMD 102, 104, it can be said that the output(s) of the accelerometer 154 are indicative of an orientation of the LIMD 102, 104. More specifically, in accordance with certain embodiments, the controller 112 of an LIMD 102, 104 receives one or more output(s) of the accelerometer 154, which is/are indicative of an orientation of the LIMD 102, 104. In such embodiments, the controller 112 can determine, based on the output(s) received from the accelerometer 154, an actual orientation of the LIMD 102, 104. Each output of the accelerometer 154 can comprise a respective signal.

One or more signals produced and output by the accelerometer 154 may be analyzed with respect to frequency content, energy, duration, amplitude and/or other characteristics. Such signals may or may not be amplified and/or filtered prior to being analyzed. For example, filtering may be performed using lowpass, highpass and/or bandpass filters. The signals output by the accelerometer 154 can be analog signals, which can be analyzed in the analog domain, or can be converted to digital signals (by an analog-to-digital converter) and analyzed in the digital domain. Alternatively, the signals output by the accelerometer 154 can already be in the digital domain.

The one or more signals output by the accelerometer 154 can be analyzed by the controller 112 and/or other circuitry. In certain embodiments, the accelerometer 154 is packaged along with an integrated circuit (IC) that is designed to analyze the signal(s) it generates. In such embodiments, one or more outputs of the packaged sensor/IC can be an indication of acceleration along one or more axes. In other embodiments, the accelerometer 154 can be packaged along with an IC that performs signal conditioning (e.g., amplification and/or filtering), performs analog-to-digital conversions, and stores digital data (indicative of the sensor output) in memory (e.g., RAM, which may or may not be within the same package). In such embodiments, the controller 112 or other circuitry can read the digital data from the memory and analyze the digital data. Other variations are also possible, and within the scope of embodiments of the present technology. In accordance with certain embodiments of the present technology, described in additional detail below, a sensor signal produced by the accelerometer 154 of an LIMD 102, 104 implanted in or on a cardiac chamber can be used to detect mechanical cardiac activity associated with another cardiac chamber.

Figure 1B depicts a single LIMD 102, 104 and shows the LIMD's functional elements substantially enclosed in a hermetic housing 110. The LIMD 102, 104 has at least two electrodes 108 located within, on, or near the housing 110, for delivering pacing pulses to and sensing electrical activity from the muscle of the cardiac chamber, and for bidirectional communication with at least one other device within or outside the body. Hermetic feedthroughs 130, 131 conduct electrode signals through the housing 110. The housing 110 contains a primary battery 114 to supply power for pacing, sensing, and communication. The housing 110 also contains circuits 132 for sensing cardiac activity from the electrodes 108, one or more receivers 120, 122 for receiving information from at least one other device via the electrodes 108, and the pulse generator 116 for generating pacing pulses for delivery via the electrodes 108 and also for transmitting information to at least one other device via the electrodes 108. The housing 110 can optionally contain circuits for monitoring device health, for example a battery current monitor 136 and a battery voltage monitor 138 can contain circuits for controlling operations in a predetermined manner.

In Figure 1B, all of the components shown within the housing 110, besides the battery 114, can be referred to generally as electrical circuitry or electronics of the LIMD 102, 104. In Figure 1B the battery 114 and the electronics are shown as being within the same housing 110. In some embodiments, such as in Figure 2B, the battery 114 and the electronics are included within separate respective electrically conductive housings (e.g., 220 and 222 in Figure 2B) that are electrically isolated from one another.

The electrodes 108 can be configured to communicate bidirectionally among the multiple LIMDs 102, 104 to coordinate pacing pulse delivery and optionally other therapeutic or diagnostic features using messages that identify an event at an individual LIMD originating the message and an LIMD receiving the message react as directed by the message depending on the origin of the message. An LIMD 102, 104 that receives the event message reacts as directed by the event message depending on the message origin or location. In some embodiments or conditions, the two or more leadless electrodes 108 can be configured to communicate bidirectionally among the one or more LIMDs 102, 104 and transmit data including designated codes for events detected or created by an individual LIMD. Individual LIMDs can be configured to issue a unique code corresponding to an event type and a location of the sending pacemaker. While the LIMD 102, 104 shown in Figure 1B is shown as including only two electrodes 108, in alternative embodiments discussed below, an LIMD can include more than two electrodes.

In some embodiments, an individual LIMD 102, 104 can be configured to deliver a pacing pulse with an event message encoded therein, with a code assigned according to pacemaker location and configured to transmit a message to one or more other LIMDs via the event message coded pacing pulse. The pacemaker or pacemakers receiving the message are adapted to respond to the message in a predetermined manner depending on type and location of the event.

LIMD 102, 104 can be configured for operation in a particular location and a particular functionality at manufacture and/or at programming by an external device 109 (e.g., programmer, remote programming engine). Bidirectional communication among the multiple leadless cardiac pacemakers can be arranged to communicate notification of a sensed heartbeat or delivered pacing pulse event and encoding type and location of the event to another implanted pacemaker or pacemakers. The LIMD 102, 104 receiving the communication decodes the information and responds depending on location of the receiving pacemaker and predetermined system functionality.

In some embodiments, the LIMDs 102, 104 are configured to be implantable in any chamber of the heart, namely either atrium (RA, LA) or either ventricle (RV, LV). Furthermore, for dual-chamber configurations, multiple LIMDs 102, 104 may be co-implanted (e.g., one in the RA and one in the RV, or one in the RV and one in the coronary sinus proximate the LV). Certain pacemaker parameters and functions depend on (or assume) knowledge of the chamber in which the pacemaker is implanted (and thus with which the LIMD is interacting, e.g., pacing and/or sensing). Some non-limiting examples include: sensing sensitivity, an evoked response algorithm, use of AF suppression in a local chamber, blanking and refractory periods, etc. Accordingly, each LIMD preferably knows an identity of the chamber in which the LIMD is implanted, and processes may be implemented to automatically identify a local chamber associated with each LIMD.

Also shown in Figure 1B, the primary battery 114 has positive pole 140 and negative pole 142. Current from the positive pole 140 of the primary battery 114 flows through an optional shunt 144 to an optional regulator circuit 146 to create a positive voltage supply 148 suitable for powering the remaining circuitry of the LIMD 102, 104. The optional shunt 144 enables the optional battery current monitor 136 to provide the controller 112 with an indication of battery current drain and indirectly of device health. The illustrative power supply can be a primary battery 114.

In certain embodiments, the electrodes 108 of an LIMD 102, 104 can be used to sense an intracardiac electrocardiogram (IEGM) from which atrial and/or ventricular activity can be detected, e.g., by detecting R-waves and/or P waves. Accordingly, the sensed IEGM can be used by an LIMD 102, 104 to time its delivery of pacing pulses. Where an IEGM sensed by an LIMD 102, 104 is indicative of electrical cardiac activity associated with the same cardiac chamber within or on which an LIMD 102, 104 is implanted (e.g., the local chamber of the heart), the IEGM can be referred to as a near-field (NF) signal. Where an IEGM sensed by an LIMD 102, 104 is indicative of electrical cardiac activity associated with a remote cardiac chamber of the heart (other than the cardiac chamber within or on which the LIMD 102, 104 is implanted), the IEGM can be referred to as a far-field (FF) signal. An IEGM can also be used by an LIMD 102, 104 to time when i2i communication pulses should be generated and transmitted.

Similarly, the HS sensor (e.g., accelerometer 154) can sense HS signals that are generally associated with cardiac valve closures, such as S1, S2, etc.

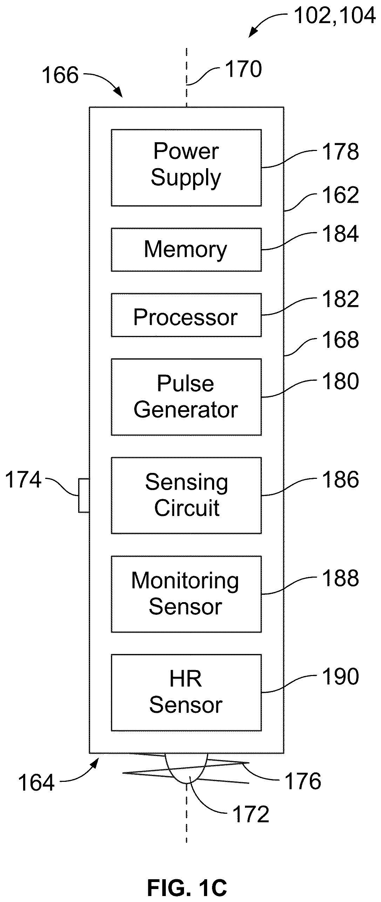

Figure 1C illustrates a side view of the LIMD 102, 104 according to embodiments herein. The illustrated LIMD 102, 104 includes a schematic representation of some internal components of the LIMD 102, 104. The housing 162 of the LIMD 102, 104 includes a first mounting end 164, an opposite second end 166, and an intermediate shell 168 extending between the first end 164 and the second end 166. The shell 168 is elongated and tubular in shape and extends along a longitudinal axis 170. The mounting end 164 mounts to tissue of an intra-cardiac wall within a chamber of the heart.

The mounting end 164 includes an electrode 172 securely attached thereto and projecting outward from the mounting end 164. The shell 168 includes one or more electrodes 174 provided therein remote from the electrode 172. The electrodes 172, 174 cooperate to define a sensing vector and to sense local and remote CA signals. The electrodes 172, 174 are further configured to deliver stimulation energy to tissue of interest. As used herein, "tissue of interest" refers to intra-cardiac tissue that the LIMD 102, 104 is configured to monitor and provide stimulation energy. In the illustrated embodiment, the LIMD 102, 104 is configured to be affixed directly to the tissue of interest, as described below. The electrode 172 may be a cathode electrode that is actively fixated to the myocardium, while the electrode 174 is an anode electrode. The stimulation energy may be in the form of low-energy pacing pulses.

When the mounting end 164 is mounted to the intra-cardiac tissue, the electrode 172 is securely affixed to and engages the tissue of interest to deliver the stimulation energy directly thereto. In addition to delivering stimulation energy, in an alternative embodiment the electrode 172 may be used to sense electrical activity from the tissue of interest. The electrode 172 may be formed as a single conductive bulb or, alternatively, as a cone, a helix, a single wire, or the like. Optionally, the electrode 172 is not covered with insulation material and the conductive material is exposed to facilitate a good electrical connection to the local wall tissue. Alternatively, at least a portion of the electrode 172 is covered with insulation to prevent electrical conduction to tissue that engages the insulation.

The mounting end 164 includes a fixation element 176 to secure the LIMD 102, 104 in or proximate to a local chamber of the heart. For example, the fixation element 176 may be a fixation screw 176 securely attached thereto and projecting outward from the mounting end 164. The fixation screw 176 is configured to extend into the tissue of interest to anchor the LIMD 102, 104 to the intra-cardiac tissue. The fixation screw 176 is configured to be screwed into the tissue to firmly adhere the LIMD 102, 104 thereto by pressing the mounting end 164 against the tissue and rotating the LIMD 102, 104 in a first, coupling direction. The fixation screw 176 may be extracted from the tissue by rotating the LIMD 102, 104 in an opposite, uncoupling direction in conjunction with a slight tugging force directed away from the myocardial wall. The fixation screw 176 may be shaped as a helical corkscrew that defines a center channel. For example, the fixation screw 176 may surround the electrode 172 such that the electrode 172 is within the center channel. In an alternative embodiment, the fixation screw 176 is part of the electrode 172. For example, the electrode 172 may have helical threads on an outer surface of the electrode 172, such that the electrode 172 forms the fixation screw 176.

Additionally or alternatively, the fixation element may include one or more loops, tabs, tines, and the like that are configured to retain the LIMD 102, 104 in a vessel, septal wall, or other tissue proximate to the local chamber of the heart. For example, the LIMD 102. 104 and/or fixation element may be formed as described in one or more of U.S. Pat. Pub. No. 2019/0099087, publishing April 4, 2019, and titled WIRELESS SENSOR FOR MEASURING PRESSURE; U.S. Patent 9,993,167, issuing June 12, 2018 and titled APPARATUS AND METHOD FOR SENSOR DEPLOYMENT AND FIXATION; U.S. Published Application 2016/0007924, publishing January 14, 2016, and titled IMPLANTABLE PRESSURE TRANSDUCER SYSTEM OPTIMIZED TO CORRECT ENVIRONMENTAL FACTORS, and U.S. Pat. Pub. No. 2012/0172690, filed October 28, 2011, and titled IMPLANTABLE MEDICAL DEVICE FIXATION.

The housing 162 retains a power supply 178 and various electronic components that receive electrical current from the power supply 178. The electronic components provide the functionality of the LIMD 102, 104, such as controlling the stimulation energy delivered to the electrode 172 and sensing the depolarization along the tissue of interest in response to a pacing pulse or to an intrinsic heartbeat. The power supply 178 stores charge for gradual disbursal to the electronic components as needed. The power supply 178 may be a battery, such as the primary battery 114 in Figure 1B. The power supply 178 has a fixed amount of charge at full capacity. The power supply 178 may be rechargeable in some embodiments and may not be rechargeable in other embodiments. The power supply 178 is fully retained within and surrounded by the housing 162.

The electronic components include a pulse generator 180, such as pulse generator 116 in Figure 1B, a processor 182, such as controller 112 in Figure 1B, a memory 184, a sensing circuit 186, such as sensing circuit 132 in Figure 1B, and a monitoring sensor 188, such as accelerometer 154 in Figure 1B. The illustration is intended as an overview of the electronic components only according to an embodiment of the LIMD 102, 104. The pulse generator 180 provides stimulation energy to the electrode 172 which is delivered to the tissue of interest that the electrode 172 engages. The pulse generator 180 includes circuitry to control the output of stimulation energy directed to the electrode 172. For example, the pulse generator 180 produces lower energy pulses for pacing.

The processor 182 is a controller that controls the flow of charge between the power supply 178, the electronic components (such as the pulse generator 180, monitoring sensor 188 and the sensing circuit 186), and the electrodes (such as electrode 172). For example, the processor 182 controls the timing and intensity or magnitude of the stimulation pulses. If multiple electrodes are used to deliver stimulation energy to the intra-cardiac tissue, the processor 182 may synchronize the delivery of the pulses. The processor 182 is communicatively coupled to the pulse generator 180, the sensing circuit 186, the memory 184, and the power supply 178. The processor 182 also functions based on instructions stored locally in the memory 184. The memory 184 is a non-transitory tangible computer readable storage medium. The memory 184 stores programmable and executable instructions (e.g., program instructions) for the processor 182. The processor 182 is responsive to the programmable instructions to control operation of the LIMD 102, 104 as described herein. The memory 184 may also store data. Some of the data may be stored prior to completing assembly of the LIMD 102, 104, while other data may be stored during use of the implanted LIMD 102, 104. For example, the memory 184 may be used to store data on intrinsic electrical activity within the heart as monitored by the sensing circuit 186, data on the number, time, and/or magnitude of pacing pulses generated by the pulse generator 180, or the like. The memory 184 is further configured to store NF and FF CA signals, HS signals, atrial events, ventricular events, characteristics of interest (COIs) that are identified and associated with particular signals, surrogate AV intervals that are determined, alerts that are generated, criteria (which may include threshold(s) for evaluating trending statistics based on surrogate AV intervals over time, monitoring frequency to determine surrogate AV intervals, machine learning models, and the like.

The sensing circuit 186 is configured to monitor intrinsic electrical CA signals within the local chamber of the heart and FF CA signals associated with a remote chamber of the heart. The sensing circuit 186 is communicatively coupled to one or more sensing electrodes 172, 174 located on or extending from the housing 162. The sensing electrodes 174 are shown located along one or more sides of the shell 168 but may additionally or alternatively be located along the second end 166 of the housing 162. The sensing electrode 174 senses electrical activity, such as physiologic and pathologic behavior and events, and provides sensed signals to the sensing circuit 186 in response. In an alternative embodiment, the pulsing electrode 172 doubles as a sensing electrode, such that the pulsing electrode 172 is used to deliver stimulation pulses and, in-between pulses, monitors the electrical activity within the tissue of interest for the sensing circuit 186.

Although not explicitly shown, the LIMD 102, 104 can include one or more of wireless transceivers, communication coils and antenna, and/or use conductive communication to communicate with other LIMDs, other implantable devices, the external device 109, the monitor 158, and medication delivery device 160, and the like as discussed in Figure 1A. In some cases, the one or more processor 182 can wirelessly transmit at least one of the CA signals, the COIs, the FF CA signals, the HS signals, the atrial events, the ventricular events, the surrogate AV intervals, generated diagnostic data, or the alert(s) to the external device 109, 158, 160.

In some embodiments, the external device 109 includes memory to store program instructions, and one or more processors that, when executing the program instructions, can receive or collect an electrocardiogram (ECG) associated with the heart, determine COIs associated with, for example, R-waves within the ECG, and determine surrogate AV intervals between the atrial events received from the LIMD and associated COIs.

The criteria for evaluating trending statistics are programmable and can be stored in the memory 184. In some cases, the external device 109 or other external device can remotely program or update the criteria, and/or the LIMD 102, 104 can adjust the criteria based on predetermined data, thresholds, and the like.

Optionally, the LIMD 102, 104 may include a heart rate (HR) sensor 190 configured to obtain HR data indicative of a patient's heart rate. For example, the HR sensor 190 may sense a blood temperature indicative of a core body temperature of the patient.

In some embodiments, the one or more processors 182 further generate a second alert in response to i) a second one of the time intervals or ii) a second plurality of the time intervals satisfying a second threshold, wherein the second threshold is indicative of a second increase in the delay. In other embodiments, the atrial events comprise at least one of atrial pace (AP) events or atrial sense (AS) events.

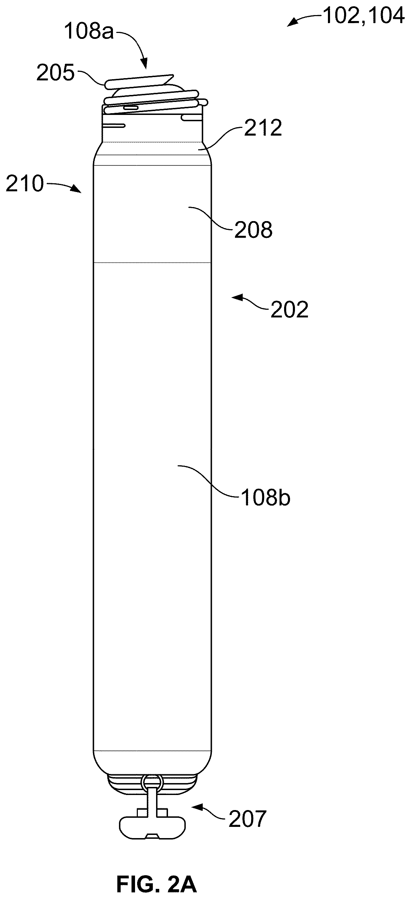

Figure 2A shows an LIMD 102, 104. The LIMD 102, 104 can include a hermetic housing 202 (e.g., the housing 110 in Figure 1B or the housing 162 in Figure 1C) with electrodes 108a, 108b disposed thereon. As shown, electrode 108a can be separated from but surrounded partially by a fixation mechanism 205, and the electrode 108b can be disposed on the housing 202. In other embodiments, the fixation mechanism 205 can also function as an electrode. The fixation mechanism 205 can be a fixation helix, a spear, a plurality of hooks, barbs, or other attaching features configured to attach the LIMD 102, 104 to tissue, such as heart tissue. In some cases, the fixation mechanism 205 can have a plurality of tines that can be formed of a shape memory material, such as Nitinol. The plurality of tines can be bent elastically to secure the LIMD 102, 104, such as from a more straight position to a hooked position, allowing the times to bend again to the more straight position if the LIMD 102, 104 is to be repositioned. The electrodes 108a, 108b are examples of the electrodes 108 shown in and discussed above with reference to Figure 1B and the electrodes 172, 174 in Figure 1C. One of the electrodes 108 (e.g., 108a) can function as a cathode type electrode and another one of the electrodes 108 (e.g., 108b) can function as an anode type electrode, or vice versa, when the electrodes are used for delivering stimulation. The electrode 108a is an example of a tip electrode, and the electrode 108b is an example or a ring electrode. The electrodes 108a, 108b can be referred to collectively as the electrodes 108, or individually as the electrode 108. While the LIMD 102, 104 shown in Figure 2A is shown as including only two electrodes 108, in alternative embodiments discussed below, an LIMD can include more than two electrodes. The LIMD 102, 104 shown in Figure 2A is also shown as including a retrieval feature 207, which can include a "button" or circular grasping feature that is configured to dock within a docking cap or a retrieval catheter that can be used to remove the LIMD 102, 104 when it needs to be removed and/or replaced. Alternative form factors for the retrieval feature are also possible.

Where an LIMD includes more than two electrodes, a first subset of the electrodes can be used for delivering pacing pulses, a second subset of the electrodes can be used for sensing a near-field signal, a third subset of the electrodes can be used for sensing a far-field signal, and a fourth subset of the electrodes can be used for transmitting and receiving i2i messages. One or more of the first, second, third, and forth subsets of electrodes can be the same, or they can all differ from one another. As used herein, the term near-field signal refers to a signal that originates in a local chamber (i.e., the same chamber) within which or on which corresponding sense electrodes (and the LIMD including the sense electrodes) are located. Conversely, the term far-field signal refers to a signal that originates in a chamber other than the local chamber within which or on which corresponding sense electrodes (and the LIMD including the sense electrodes) are located.

The housing 202 can also include an electronics compartment 210 within the housing that contains the electronic components necessary for operation of the LIMD 102, 104, including, e.g., a pulse generator, receiver, and a processor for operation. The hermetic housing 202 can be adapted to be implanted on or in a human heart, and can be cylindrically shaped, rectangular, spherical, or any other appropriate shapes, for example.

The housing 202 can comprise a conductive, biocompatible, inert, and anodically safe material such as titanium, 316L stainless steel, or other similar materials. The housing 202 can further comprise an insulator 208 disposed on the conductive material to separate electrodes 108a, 108b. The insulator 208 can be an insulative coating on a portion of the housing between the electrodes, and can comprise materials such as silicone, polyurethane, parylene, or another biocompatible electrical insulator commonly used for implantable medical devices. In the embodiment of Figure 2A, a single insulator 208 is disposed along the portion of the housing 202 between electrodes 108a, 108b. In some embodiments, the housing 202 itself can comprise an insulator instead of a conductor, such as an alumina ceramic or other similar materials, and the electrodes can be disposed upon the housing.

As shown in Figure 2A, the LIMD 102, 104 can further include a header assembly 212 to isolate electrodes 108a, 108b. The header assembly 212 can be made from PEEK, tecothane or another biocompatible plastic, and can contain a ceramic to metal feedthrough, a glass to metal feedthrough, or other appropriate feedthrough insulator as known in the art.

The electrodes 108a, 108b can comprise pace/sense electrodes, or return electrodes. A low-polarization coating can be applied to the electrodes, such as sintered platinum, platinum-iridium, iridium, iridium-oxide, titanium-nitride, carbon, or other materials commonly used to reduce polarization effects, for example. In Figure 2A, electrode 108a can be a pace/sense electrode and electrode 108b can be a return electrode. The electrode 108b can be a portion of the conductive housing 202 that does not include an insulator 208. As noted above, and described in additional detail below, an LIMD can include more than two electrodes, and may use different combinations of the electrodes for sensing a NF signal, sensing a FF signal, delivering pacing pulses, and sending and receiving i2i messages. When the electrode 108a is used as a pace electrode it can also be referred to as the cathode.

Several techniques and structures can be used for attaching the housing 202 to the interior or exterior wall of the heart. A helical fixation mechanism 205, can enable insertion of the device endocardially or epicardially through a guiding catheter. A torqueable catheter can be used to rotate the housing and force the fixation device into heart tissue, thus affixing the fixation device (and also the electrode 108a in Figure 2A) into contact with stimulable tissue. Electrode 108b can serve as an indifferent electrode (also referred to as the anode) for sensing and pacing. The fixation mechanism 205 may be coated partially or in full for electrical insulation, and a steroid-eluting matrix may be included on or near the device to minimize fibrotic reaction, as is known in conventional pacing electrode-leads.

Figure 2B illustrates an LIMD 214 having three electrodes 234, 240, 242 in accordance with embodiments herein. The LIMD 214 is shown as including two separate housings 220, 222, each of which is made of an electrically conductive material. The housings 220, 222 can be made of the same type of electrically conductive material as one another, or of different types of electrically conductive materials than one another. The electrically conductive material of which the housing 220 and/or the housing 222 is made can be an electrically conductive biocompatible metal or alloy, such as stainless steel, a cobalt-chromium alloy, titanium, or a titanium alloy, but is not limited thereto. It would also be possible for the electrically conductive material of which the housing 220 and/or the housing 222 is made to be a currently developed or future developed electrically conductive polymeric material. Since the housings 220, 222 are each made of an electrically conductive material, they can also be referred to as electrically conductive housings 220, 222.

Electronic circuitry 224 (also referred to as electronics) is included within the housing 220, and a battery 226 (e.g., the battery 114 in Figure 1B) is located within the housing 222. The electronic circuitry 224 can include, e.g., one or more pulse generators, one or more sense amplifiers, switches, a controller, memory, and/or the like. Such a controller can include one or more processors, and/or an application-specific integrated circuit (ASIC), but is not limited thereto. Exemplary details of the electronic circuitry 224 were discussed above with reference to Figures 1B and 1C. Since the electronic circuitry 224 can likely be made smaller in size than the battery 226 (which should preferably power the LIMD 214 for a few years), it is likely that the housing 222 which encases the battery 226 is larger in volume than the housing 220 which encases the electronic circuitry 224. The housings 220, 222 are preferably hermetic housings that protect the electronic circuitry 224 and the battery 226 from the harsh environment of the human body.

An inter-housing insulator 228 is located between the housing 220 and the housing 222 to electrically isolate the housings 220 and 222 from one another. Conductors 230, 232 that extend through the inter-housing insulator 228 connect the positive (+) and negative (-) poles of the battery 226 to the electronics 224, which are encased within the electrically conductive housing 220, to thereby enable the battery 226 to provide power to the electronics 224.

The LIMD 214 is shown as including a tip electrode 234 that is located adjacent to the free end of the housing 220. The tip electrode 234 is electrically isolated from the electrically conductive housing 220 by an insulator 236. A feedthrough 238 that extends through the insulator 236 is used to connect the tip electrode 234 to the electronics 224 (e.g., one or more pulse generators and/or one or more sense amplifiers). The tip electrode 234 can have various different shapes, depending upon implementation. For example, the tip electrode 234 can have an annular shape, a semi-spherical cap, or a helical shape to enable the tip electrode 234 to also function as an attachment mechanism for attaching the LIMD 214 to an interior or exterior wall of a cardiac chamber. Where the tip electrode 234 has a helical shape it can also be referred to as a helical or helix electrode. Other shapes for the tip electrode 234 are also possible and within the embodiments of the present technology described herein.

The LIMD 214 a is also shown as including a ring electrode 240 and a ring electrode 242. In certain embodiments, the ring electrode 240 is provided by a non-insulated portion of the electrically conductive housing 220. More specifically, portions of the electrically conductive housing 220 can be coated or otherwise covered by an insulator 244, and a non-insulated portion of the housing 220 can provide the ring electrode 240. Similarly, the ring electrode 242 can be provided by a non-insulated portion of the electrically conductive housing 222. More specifically, portions of the electrically conductive housing 222 can be coated or otherwise covered by an insulator 246, and a non-insulated portion of the housing 222 can provide the ring electrode 242. Such insulators 244, 246 can be made of various different types of biocompatible insulating materials, such as, but not limited to, ceramic, polyurethane, parylene, or silicone.

An advantage of having the inter-housing insulator 228 electrically isolate the housings 220, 222 from one another is that the ring electrodes 240, 242 can be used independently of one another. Further, it is noted that designing an LIMD 214 to include three electrodes 234, 240, 242 provides an advantage of enabling better sensing of FF signals, compared to if the LIMD included only two electrodes.

In accordance with certain embodiments, during pacing of a cardiac chamber (e.g., RA chamber or RV chamber) within or on which the LIMD 214 is implanted, the tip electrode 234 is connected as the cathode and one of the ring electrodes 240 or 242 is connected as the anode. In other words, a tip-to-ring pacing vector can be used for pacing. It would also be possible that when performing pacing using the tip electrode 234 as the cathode, both ring electrodes 240 and 242 can be connected as the anode (e.g., a distributed anode) at the same time. In accordance with certain embodiments, sensing can be performed using one or more pair of the electrodes 234, 240, and 242. Additionally, i2i communication can be performed using a pair of the electrodes 234, 240, and 242.

In an embodiment, if the LIMD 214 is implanted within the RV chamber, then NF sensing (of electrical cardiac activity associated with the RV chamber) can be performed using the tip electrode 234 and the ring electrode 240. In other words, a tip-to-ring sensing vector can be used for NF sensing. In an embodiment, if the LIMD 214 is implanted in the RV chamber, then FF sensing (of electrical cardiac activity associated with the RA chamber) can be performed using the tip electrode 234 and the ring electrode 242, since the ring electrode 242 will be the electrode closest to the RA chamber. In other words, a separate tip-to-ring sensing vector can be used for FF sensing than is used for NF sensing. In still another embodiment, if the LIMD 214 is implanted in the RV chamber, FF sensing (of electrical cardiac activity associated with the RA chamber) can be performed using the ring electrode 240 and the ring electrode 242. In other words, a ring-to-ring sensing vector can be used for FF sensing. In other embodiments, the two electrodes that maximize the FF cardiac activity signal relative to the atrial events can be selected. The tip electrode 234 and the ring electrode 242 can be used for i2i communication (both transmitting of i2i pulses and receiving of i2i pulses) with another LIMD, another IMD, and/or with an external device (e.g., programmer). Alternatively, the tip electrode 234 and the ring electrode 240 can be used for i2i communication (both transmitting of i2i pulses and receiving of i2i pulses) with another LIMD, another IMD, and/or with an external device. In still other embodiments, the ring electrode 240 and the ring electrode 242 can be used for i2i communication (both transmitting of i2i pulses and receiving of i2i pulses) with another LIMD, another IMD, and/or with an external device.

In an embodiment, if the LIMD 214 is implanted within the RA chamber, then NF sensing (of electrical cardiac activity associated with the RA chamber) can be performed using the tip electrode 234 and the ring electrode 240 (i.e., a tip-to-ring sensing vector can be used for NF sensing); FF CA sensing (of electrical cardiac activity associated with the RV chamber) can be performed using the tip electrode 234 and the ring electrode 242 (since the ring electrode 242 will be the electrode closest to the RV chamber) or using the ring electrode 240 and the ring electrode 242 as these electrodes are closest to the RV chamber; and the tip electrode 234 and one of the ring electrodes 240 or 242 can be used for i2i communication (both transmitting of i2i pulses and receiving of i2i pulses) with another LIMD, another IMD, and/or with an external device, or the two ring electrode 240 and 242 can be used for i2i communication.

More generally, when the LIMD 214 is implanted within a cardiac chamber, NF sensing (of electrical cardiac activity associated with the local chamber within which the LIMD 214 is implanted) can be performed using the tip electrode 234 and the ring electrode 240 (i.e., a tip-to-ring sensing vector can be used for NF sensing); FF sensing (of electrical cardiac activity associated with a remote chamber) can be performed using the tip electrode 234 and the ring electrode 242, or using the ring electrode 240 and the ring electrode 242; and i2i communication can be performed using the tip electrode 234 and one of the ring electrodes 240 or 242, or using both ring electrode 240 and 242. Other variations are also possible and within the scope of the embodiments of the present technology.

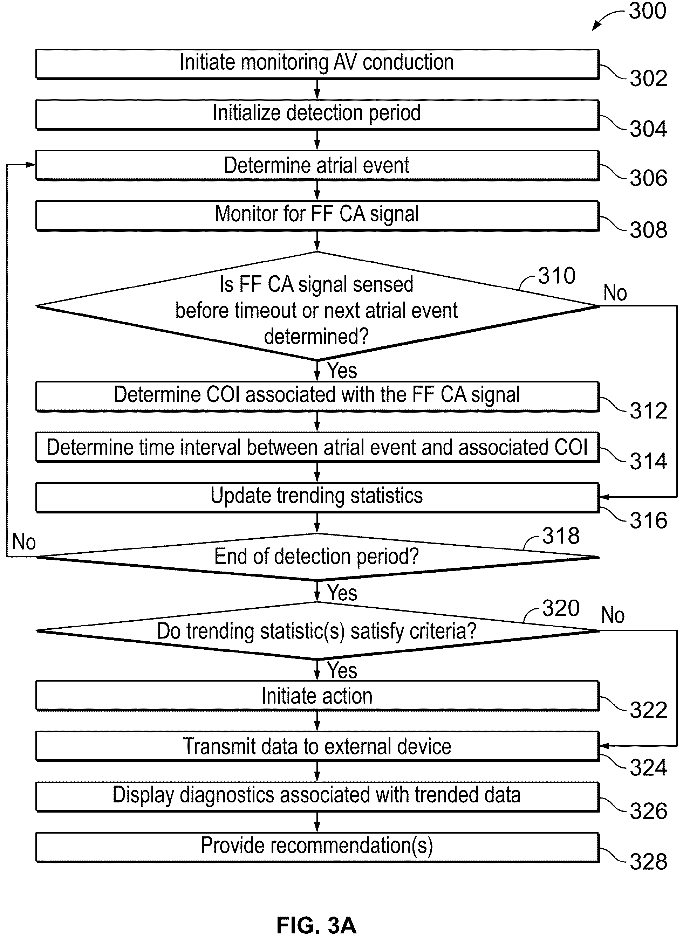

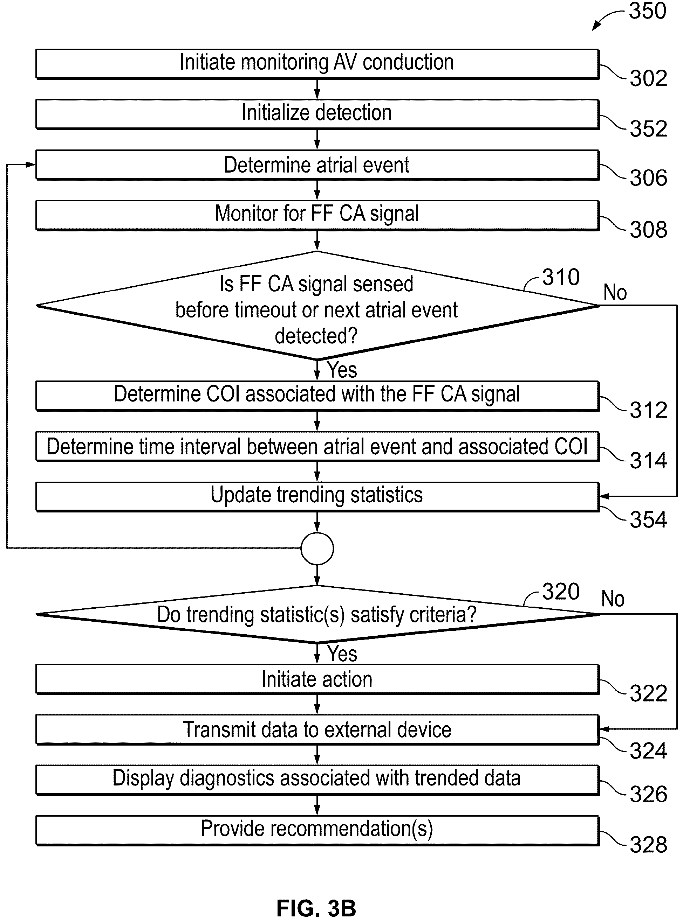

Figures 3A and 3B illustrate computer-implemented and/or processor-implemented methods 300 and 350, respectively, that monitor AV conduction in a patient and control pacing therapy of the patient. The method can utilize an LIMD 102 implanted in, within, or on a chamber of the heart to sense CA signals of the local chamber (e.g., NF CA signals) and FF CA signals associated with a remote chamber. In some embodiments, the LIMD 102 can be implanted in the RA. Time intervals can be determined between atrial events associated with the local chamber and COIs associated with the FF CA signals, and trending statistics can be updated. In response to the trending statistics indicating an increase in the surrogate AV intervals or increase in AV conduction disease, an alert can be generated or an operational mode of an implanted LIMD 102 can be changed, among other actions discussed below. In other embodiments, the LIMD 104 can be implanted in a ventricle.

The operations of Figures 3A and 3B may be implemented by hardware, firmware, circuitry and/or one or more processors housed partially and/or entirely within an LIMD 102, an external device 109, wearable device, a local external device, remote server or more generally within a health care system. Optionally, the operations of Figures 3A and 3B may be shared across devices, and thus partially implemented by one or more of an LIMD 102, an external device 109, wearable device, a local external device, remote server or more generally within a health care system. For example, the LIMD 102 includes IMD memory and one or more IMD processors, the external device 109 includes external device memory and one or more external device processors, and further, other external devices/systems (e.g., local, remote or anywhere within the health care system) that may implement the operations of Figures 3A and 3B include external device memory and one or more external device processors.

Although the methods of Figures 3A and 3B are written primarily from the viewpoint of the LIMD 102 implanted in the RA, it should be understood that after signal data, such as the CA signals, including NF and FF CA signals as well as atrial events, are sensed or determined, the signal data can be transmitted to an external device or a different implanted device for evaluation/processing/storage.

Turning first to Figure 3A, at 302, one or more processors initiate monitoring of AV conduction within a heart of a patient and select electrodes of the LIMD 102 for sensing. The surrogate AV interval can be measured at predetermined intervals or monitoring frequency, such as hourly, daily, weekly, monthly, etc. Optionally, the measurements can be generated when a patient is at rest or active, based on a clock time, detected sleep state, activity of the patient, posture of the patient (prone, sitting, standing, etc.). In still further embodiments, the measurements can be generated on demand, such as via a command received from an external device.

In some embodiments, a single LIMD 102 can be implanted in or on the RA, which can be referred to as the local chamber. The LIMD 102 also senses FF CA signals associated with the ventricles. At 302, in some embodiments, if the LIMD 102 includes more than two electrodes, the one or more processors can select an electrode pair for detecting the FF CA signals that maximizes the FF CA signal relative to the NF CA signal, such as electrodes 240 and 242 of Figure 2B. For example, the level of the FF CA signals detected from a remote chamber of the heart can be improved by selecting the electrode pair closest to the remote chamber. In other embodiments, a different electrode pair can be identified for the detection of NF CA signals, such as those closest to the RA (e.g., electrodes 234 and 240). In further embodiments, even if the LIMD 102 includes more than two electrodes, the same two electrodes can be selected to detect both the NF and FF CA signals.

At 304, the one or more processors initialize a detection period over which to sense the CA signals for a series of heartbeats. The detection period can be a predetermined time period, such as 10 seconds, 30 seconds, one minute, two minutes, etc. Alternatively, the detection period can be based on a predetermined number of heartbeats or a predetermined number of atrial events.

At 306, the one or more processors determine an atrial event, such as a first atrial event. The atrial events can be atrial pace (AP) events and/or atrial sense (AS) events. The one or more processors continue to monitor to determine the next or second atrial event.

At 308, the one or more processors monitor, using the electrodes of the LIMD, for the FF CA signal that includes a signal indicative of ventricular activity.

At 310, the one or more processors determine whether the FF CA signal is sensed before the next atrial event is determined or before a timeout window expires. In some cases, the timeout window can be set, such as by the physician, and may be based on the patient's previously determined surrogate AV interval, measured intrinsic AV interval, and/or determined A-A interval (e.g., averaged over a predetermined time). If the FF CA signal is sensed, flow passes to 312. If no FF CA signal is sensed before the timeout window expires, or a next atrial event is determined, flow passes to 316.

At 312, the one or more processors determine a COI associated with the FF CA signal. For example, the COI can be a peak or start of the FF R-wave (e.g., Far-R-wave or signal). It should be understood that other COIs may be determined.

At 314, the one or more processors determine a time interval between the atrial event (e.g., the first atrial event (e.g., AP, AS) determined at 306) and the COI determined at 312. For example, the time interval is the surrogate AV interval (e.g., surrogate measurement of intrinsic AV delay).

At 316, the one or more processors generate diagnostic data by updating trending statistics associated with the surrogate AV interval. If an FF R-wave is not identified at 310 before the next atrial event, no time interval is determined for that atrial event. In some cases, this may occur when the FF R-wave is not consistently large enough to be detected by the LIMD 102 in the RA. In other cases, this may occur when no R-wave is generated within the ventricles during that cardiac cycle (such as may occur for certain AV conduction dysfunctions; for example, second- or third-degree heart block). Trending statistics can include, but are not limited to, average and/or standard deviation AV interval, minimum and maximum AV interval, average and/or standard deviation of the difference between AV intervals from consecutive cardiac cycles, and percentage of cycles without FF CA detection. Trending statistics can also include, but are not limited to, binned histograms of AV intervals and/or binned histograms of differences between AV intervals from consecutive cardiac cycles. In certain embodiments, these trending statistics may be further stratified based on whether the atrial event was a paced event or a sensed event. These trending statistics may also be stratified based on the atrial-to-atrial ("AA") interval or rate of each associated cardiac cycle. In some embodiments, the one or more processors can prepare the data for subsequent display to the clinician, such as in a chart, histogram, etc. As the surrogate AV interval data for the patient is acquired and transmitted over time (e.g., multiple days, months, quarters, years), increasing amounts of data can be trended to help inform practitioners of a patient's increase in AV delay and/or progression of AV conduction disease. In other embodiments, the preparation of data can occur later in the process.

At 318, the one or more processors determine whether the detection period initialized at 304 is complete. If no, flow returns to 306 to determine the next atrial event. If the detection period is complete, flow passes to 320. It should be understood that the trending statistics 316 can be updated to reflect data associated with all of the time intervals after the detection period of 318 is complete.

At 320, the one or more processors determine whether the trending statistics satisfy one or more criteria, such as one or more threshold, indicative of an increase in surrogate AV interval and/or progression of AV conduction disease. For example, the one or more processors can evaluate the diagnostic data generated at 316 against one or more criteria stored in the LIMD 102 or the external device 109. In some embodiments, the one or more processors can evaluate the diagnostic data by applying a machine learning model trained to classify progression of AV conduction disease. In some embodiments, the one or more criteria may include a threshold for average AV interval. The threshold may be programmable by a clinician and can be measured in, for example, milliseconds. The threshold can be based on industry standards and/or a population of patients and/or can be based on and/or relative to a patient's previously measured AV delay or determined surrogate AV interval, such as to track progression of the Av conduction disease over time, e.g., to track how quickly the patient is progressing toward total heart block. In some embodiments, a threshold of 200 milliseconds (ms), 280 ms, 300 ms, etc., can be selected. In still other embodiments, the one or more criteria may include a threshold for percentage of cycles without FF CA detection. This threshold may also be programmable by a clinician, and may provide, for example, an indicator of a patient's progression to second- or third-degree heart block. In some embodiments, a threshold of 10%, 20%, 30%, etc., may be selected. In general, the one or more criteria may include one or more thresholds associated with one or more of the trending statistics.

In some cases, more than one threshold can be used, providing data regarding the increase in surrogate AV interval, progression of AV conduction disease, or degree of heart block, which may minimize the number of times the thresholds need to be updated or customized.

A threshold can be associated with a period of time, such as a single acquisition period (e.g., the time period, short-term based) or span an extended period of time (e.g., long-term based), including trended data of previously acquired time intervals to track the increase and/or rate of increase in the surrogate AV interval and/or its variability within 3 months, 6 months, one year, and the like. This can provide the advantage of projecting how quickly a patient may be deteriorating, indicate that the patient has been stable for an extended period of time, etc. Further, different thresholds can be associated with different COIs and/or whether AS or AP are used, which may result in different surrogate measurements of AV delay.

If the trending statistic(s) satisfy one or more criteria at 320, flow passes to 322 and, optionally, the one or more processors initiate an action to generate alert(s) and/or modify an operational mode of at least one LIMD 102 to change the operational mode. Alert(s) can be saved in the memory of the LIMD 102. In some cases, the time intervals, the CA signals, atrial events, COIs, etc., can also be saved. Optionally, if more than one threshold is used, alerts may be associated with a particular threshold to provide a more detailed diagnostic to the clinician. In some embodiments, an alert can indicate that more proactive AV synchronization therapy may be appropriate (such as would be achieved with the implantation of a ventricular LIMD 104 and/or enabling of dual-chamber bradycardia therapy), and in other embodiments, an alert can indicate that the patient's condition is deteriorating and should be more frequently monitored. In still further embodiments, an alert can include at least one recommendation to i) implant a second LIMD 104 in a ventricle, ii) increase or decrease a monitoring frequency, iii) program an operational mode of one or more LIMD 102, 104 implanted in the heart 101, iv) increase or decrease the one or more thresholds, v) add, modify, and/or remove an alert, vii) add, modify, and/or discontinue a medication, viii) add, modify, and/or remove a recommendation to the patient, and ix) change a pacing setting.

At 324, optionally, the one or more processors can transmit at least one of the diagnostic data (e.g., trended data), surrogate AV intervals, CA signals, the COIs, the atrial events, action(s) and alert(s) to an external device 109 and/or another implantable device (e.g., an LIMD 104, cardiac monitor). For example, wireless transceivers, communication coils and antenna, and/or conductive communication can be used to transmit data to an external device 109 or other implanted device 104. In some embodiments, the transmission is initiated by the external device 109 or other implanted device 104. In other embodiments, the LIMD 102 can be directed to wirelessly transmit an alert and/or other data to an external device 109. It should be understood that the transmission of data, signals, etc., can occur at other times during the method, such as during the detection period, at the end of the detection period, prior to updating trending statistics, etc.

In some cases, the data may be transmitted to a patient's local device, such as their phone or other device located near their person, and then further transmitted to other external devices, such as those located in a clinician's office, on a network, etc. For example, a transceiver can be directed to wirelessly transmit an alert to an external device 109. In some cases, some or all of the applicable raw and/or processed data and signals can be transmitted to the external device. The external device 109 can activate an alert, such as a text message, instant message, email, voicemail, etc., update a patient record, etc., to alert the clinician, transmit the data/alert to other computing systems, and the like, such as to a patient care network, such as the Merlin.net^{™} patient care network operated by Abbott Laboratories (headquartered in the Abbott Park Business Center in Lake Bluff, III.).

In some embodiments, the one or more processors can transmit the alert, originally acquired data associated with the atrial and ventricular events, the surrogate AV intervals, the diagnostic data, and/or the evaluation of the diagnostic data to an external device that processes the data using artificial intelligence (AI). The external device can evaluate the diagnostic data by applying a machine learning model trained to classify progression of AV conduction disease as discussed above at 320. Further, AI can review the alert and/or history of alerts, the settings of the acquisition device, signals acquired by the acquisition device, data acquired across a cohort of similar patients, etc., and determine, based on trained machine learning models, convolutional neural networks, and the like whether the alert(s) are valid, whether adjustments to settings and/or treatments should be made, and/or generate recommendation(s) to provide to the clinician.

At 326, the one or more processors can display the diagnostics associated with the trended data (e.g., diagnostic data) to the clinician, such as in a chart, histogram, table, list, etc. The diagnostics can also display a surrogate AV interval and/or a determined or approximate degree of heart block and or classification of progression of AV conduction disease, such as in a number, percentage, graph, etc., which may in some cases be determined by a threshold, such as at 320. As the diagnostic data based on the surrogate AV intervals are generated and transmitted over time, increasing amounts of data can be trended to help inform practitioners of a patient's progression of AV delay and/or AV conduction disease. Alerts can be displayed, transmitted to designated entities, and stored.

At 328, the one or more processors can provide recommendations regarding the patient's care. Some recommendations may be provided in alerts as discussed at 322. Recommendations can include, but are not limited to, i) implant a second LIMD 104 in a ventricle, ii) increase or decrease a monitoring frequency, iii) program an operational mode of one or more LIMD 102, 104 implanted in the heart (e.g., change (e.g., reprogram) the operational mode of the LIMD 102 implanted in the RA and/or change (e.g., reprogram) the operational mode of the LIMD 104 implanted in the RV), iv) increase or decrease the one or more threshold(s), v) add, modify, and/or remove an alert, vii) add, modify, and/or discontinue a medication, viii) add, modify, and/or remove a recommendation to the patient (e.g., lifestyle, activity level), and ix) change a pacing setting. Recommendations can be displayed on a monitor, printed, added to a patient file, texted, emailed, etc.

It should be understood that some recommendations may be associated with alerts, not associated with alerts, generated by AI based on patient and/or collective data, etc. For example, AI can utilize knowledge based on many other patients with similar symptoms and/or pathology and the instant patient history to determine the appropriate settings, system configuration (e.g., how many LIMD and where implanted), monitoring frequency, operational modes, medication recommendations, etc. These recommendations provide the benefit of improving patient care and quality by providing a consistent level of care for many patients, and can decrease the amount of time medical personnel spend reviewing data, making decisions, etc.

Returning to 302, one or more processors again initiate monitoring AV conduction within the heart of the patient and select the electrodes for sensing at the predetermined intervals or monitoring frequency as described above for a second detection period. The one or more processors determine the atrial events and surrogate AV intervals over the second detection period, and update the trending of the surrogate AV intervals over time to generate updated diagnostic data.

In accordance with new and unique aspects herein, the clinician uses the measurements/data, such as the trended statistics, alerts, diagnostic data, recommendations, etc., to monitor the patient and prescribe and/or change the patient therapy (e.g., prescribe new medication, change medication, change diet, recommend physical therapy and/or activity, recommend to implant a second or different LIMD 104, change programmed parameters of LIMD(s) 102 already implanted). The patient receives improved treatment that is timely, as some of the therapies can be quickly implemented and conveyed to the patient with a phone call, text, email, etc., and/or automatically implemented electronically in real-time with or without input from the clinician. In some cases, the patient can be spared additional exams and time spent in clinic, preventing unnecessary patient stress and anxiety, and saving time and money for the patient, the medical staff, insurance companies and/or government agencies. In other cases, such as when an additional LIMD 104 is needed, the patient can be diagnosed more quickly and recommended for the procedure, improving patient outcomes.

Turning to Figure 3B, this method shares many steps with Figure 3A, which have been given the same item number and will not be further discussed in detail. In the method of Figure 3B, after the one or more processors initiate monitoring of AV conduction within a heart of a patient and select electrodes of the LIMD 102 for sensing at 302, flow passes to 352.

At 352, the one or more processors initialize detection of the sensing of NF and FF CA signals. In contrast with Figure 3A, the detection of CA signals is ongoing, such as to continue until a command to stop is received, such as from an external device 109. The collection of CA signals over an extended period of time can provide the advantage of insights that can be correlated to sleep, motion/activity, posture, as well as monitoring for an accelerated increase in the surrogate AV intervals.

The flow passes from 352 to 306, and the one or more processors determine the atrial event, monitor for the FF CA signal (308), monitor for the timeout or the next atrial event (310), determine the COI associated with the FF CA signal (312), and determine the time interval between the atrial event and the associated COI (314).

At 354, the one or more processors generate diagnostic data by updating trending statistics associated with the surrogate AV interval. In addition to the exemplary statistics that can be trended discussed above at 316, at 354 the trending statistics can be managed as moving-window statistics, exponentially filtered statistics, and the like.

From 354, flow returns to 306 as well as passing to 320.

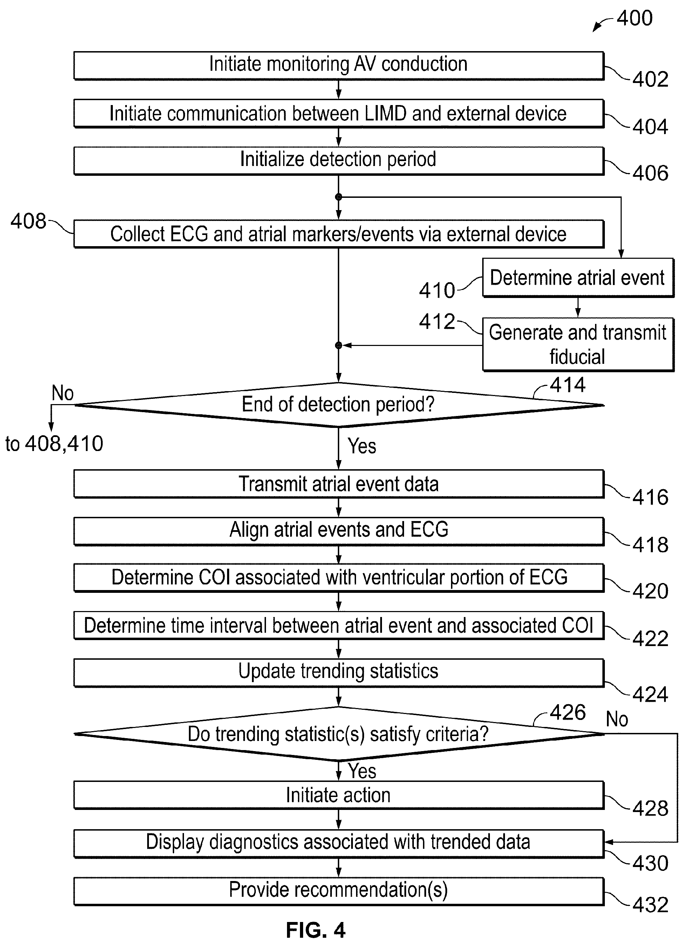

Figure 4 illustrates another computer-implemented and/or processor-implemented method 400 that, using an ECG, monitors AV conduction in a patient and controls pacing therapy of the patient. In some cases, the LIMD 102 implanted in the atrial chamber has poor detection of or is unable to detect Far R-wave, or FF CA signals associated with a remote chamber, and thus the LIMD 102 cannot reliably determine the time intervals between the atrial event and the associated COI as discussed in Figure 3A. The method of Figure 4 can utilize an LIMD 102 implanted in, within, or on a chamber of the heart to sense CA signals of the local chamber (e.g., NF CA signals). In this example, the local chamber is an atrial chamber. An external device 109 can communicate with the LIMD 102, acquiring an ECG as well as receiving the atrial events (A-pace and/or A-sense events). Time intervals can be determined between atrial events associated with the local chamber and COIs associated with the ECG, such as R-waves, VS and/or VP events, and trending statistics can be updated. In response to the trending statistics indicating an increase in the surrogate AV intervals or increase in AV conduction disease, an alert can be generated or an operational mode of an implanted LIMD 102 can be changed, among other actions discussed below.

The operations of Figure 4 may be implemented by hardware, firmware, circuitry and/or one or more processors housed partially and/or entirely within an LIMD 102, an external device 109, wearable device, a local external device, remote server or more generally within a health care system. Optionally, the operations of Figure 4 may be shared across devices, and thus partially implemented by one or more of an LIMD 102, an external device 109, wearable device, a local external device, remote server or more generally within a health care system. For example, the LIMD 102 includes IMD memory and one or more IMD processors, the external device 109 includes external device memory and one or more external device processors, and further, other external devices/systems (e.g., local, remote or anywhere within the health care system) that may implement the operations of Figure 4 include external device memory and one or more external device processors. Although the signal processing and analysis of Figure 4 is written primarily from the viewpoint of the external device, it should be understood that after signal data, such as the ECG and atrial events, are acquired, the signal data can be transmitted to another external device, the LIMD 102, or a different implanted device for evaluation/processing/storage.

At 402, one or more processors initiate monitoring of AV conduction within a heart of a patient. In some embodiments, the one or more processors select the electrodes of the LIMD 102 for sensing the atrial events as discussed above at 302. In some embodiments, a single LIMD 102 can be implanted in or on the RA, which can be referred to as the local chamber. In some embodiments, the measurements can be generated on demand, requiring the patient to interface with the external device, such as by positioning a sensor (e.g., handheld, strap, sash, garment) at an appropriate location on their body. In some cases, the external device may provide reminders to the patient depending upon the monitoring frequency. The surrogate AV intervals and/or progression of AV conduction disease can be measured at predetermined intervals or monitoring frequency, such as hourly, daily, weekly, monthly, quarterly, etc.

At 404, the one or more processors initiate communication between the LIMD 102 and the external device. In some embodiments, the processors initiate communication between the external device 109 and a second external device capable of acquiring an ECG. In other embodiments, the external device 109 communicating with the LIMD 102 is also capable of acquiring an ECG. The ECG may use lead wires and electrodes to interface with the skin of the patient.

At 406, the one or more processors initiate the detection period over which to sense the CA signals and determine the atrial events for a series of heartbeats, as well as simultaneously acquire the ECG. The detection period can be a predetermined time period, such as 10 seconds, 30 seconds, one minute, two minutes, etc. Alternatively, the detection period can be based on a predetermined number of heartbeats or a predetermined number of atrial events. The LIMD 102 and the ECG will acquire data over substantially the same period of time.

At 408, the one or more processors collect an ECG via the external device 109 for the detection period. In some embodiments, the one or more processors also collect atrial markers (e.g., AP, AS). Simultaneously, at 410 the one or more processors of the LIMD 102 determine atrial events. The atrial events can be atrial pace (AP) events and/or atrial sense (AS) events.

At 412, the one or more processors of the LIMD 102 can generate at least one fiducial that is used to align the atrial events with the ECG. The at least one fiducial can be transmitted to the external device 109 at any point during the detection period (e.g., the start of the detection period) and/or at periodic times during the detection period. In some embodiments, one or more of the atrial event(s) can be identified and used as the fiducial.

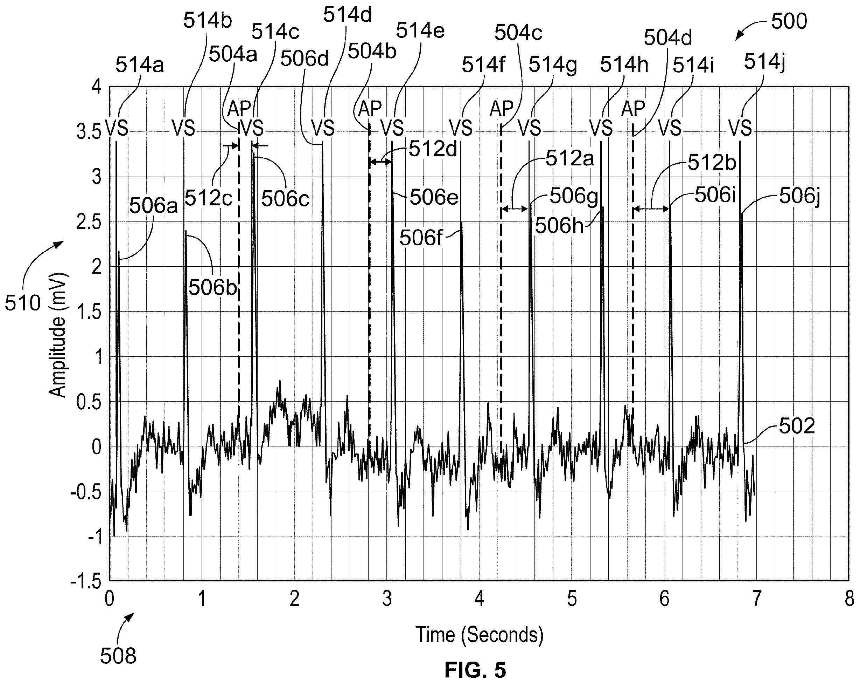

Turning to Figure 5, this figure illustrates a graph 500 of an ECG 502 and associated marker channels as acquired from the LIMD 102 by the external device 109 in accordance with embodiments herein. In this example, the LIMD 102 is implanted in the RA and the operational mode provides atrial pacing as needed. The horizontal axis 508 plots time in seconds (s) and the vertical axis 510 plots amplitude in millivolts (mV). Although only seven seconds of the ECG 502 is shown on the graph 500, it should be understood that the detection period can be longer. The graph 500 indicates the markers, such as atrial-pace (AP) events 504, and shows detected R-waves 506.

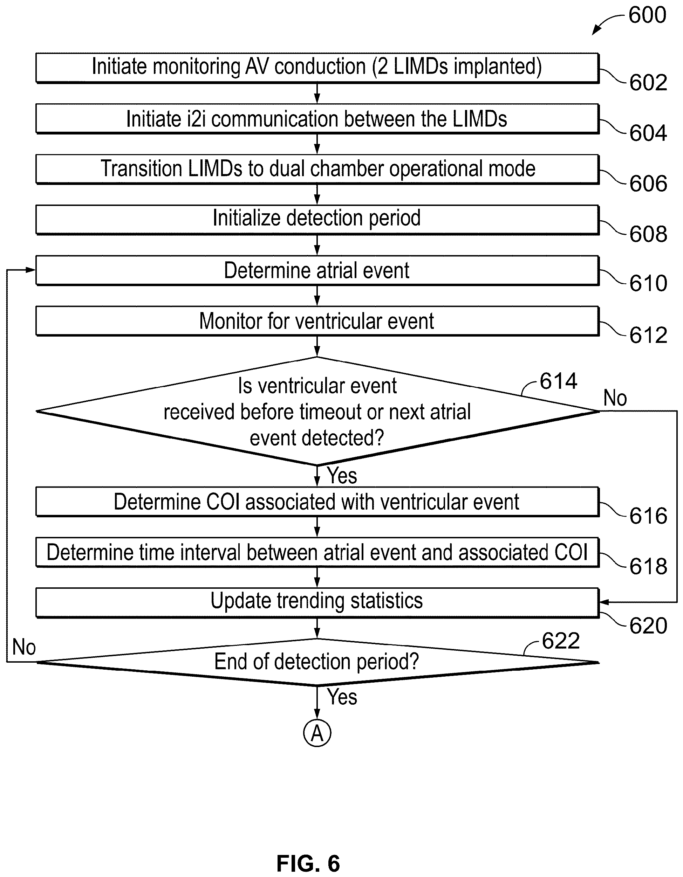

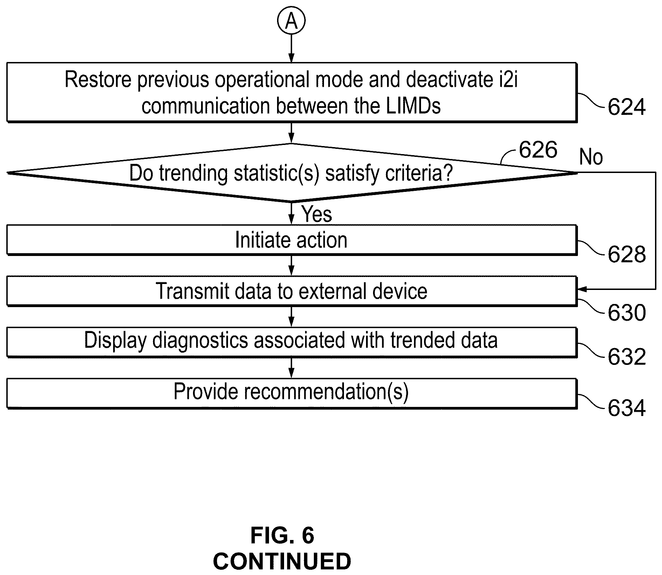

The graph 500 further indicates markers for ventricular sense (VS) events 514a-514j that are associated with the R-waves 506. Because the data for the method of Figure 4 is only collected by and from the LIMD 102 in the atrial chamber, the VS events 514 are not applicable for Figure 4 and will be discussed further below with Figure 6.

At 414 of Figure 4, the one or more processors determine whether the detection period initialized at 406 is complete. If no, flow returns to 408 and 410. If the detection period is complete, flow passes to 416.

At 416, the one or more processors transmit the atrial event data to the external device, if it has not already been transmitted.

At 418, the one or more processors align the atrial event data and the ECG, if necessary, such as by using atrial events and/or other fiducial.

At 420, the one or more processors determine a COI associated with the ventricular portion of the ECG 502. For example, the COI can be a peak or start of the R-wave 506. It should be understood that other COIs may be determined.

At 422, the one or more processors determine time intervals between the atrial events and associated COIs, providing a plurality of surrogate AV intervals (e.g., surrogate measurement of intrinsic AV delay).

For example, in Figure 5, AP event 504a is associated with R-wave 506c, AP event 504b is associated with R-wave 506e, and AP event 504c and AP event 504d are associated with R-wave 506g and R-wave 506i, respectively. Although not shown, the one or more processors has also identified the AS events (received from the LIMD 102) and associated R-wave 506. For example, each of the R-waves 506b, 506d, 506f, 506h, and 506j has an associated AS event. The R-wave 506a may not have an associated AS or AP event due to the timing of the acquisition. The one or more processors determine, over the detection period, the time between the atrial event (e.g., AP event 504, AS event) and the associated COI (e.g., peak of the associated R-wave 506, start or leading edge of the associated R-wave 506). For example, time interval 512a is determined between AP event 504c and the peak of the R-wave 506g, and time interval 512b is determined between AP event 504d and the peak of the R-wave 506i. Not all of the time intervals 512 are indicated.

At 424, the one or more processors generate diagnostic data by updating trending statistics associated with the surrogate AV intervals as discussed above at 316 and 354.

At 426, the one or more processors determine whether the trending statistics satisfy one or more criteria, such as one or more threshold, indicative of an increase in AV delay and/or progression of AV conduction disease as discussed above at 320.

If the trending statistic(s) satisfy one or more criteria at 426, flow passes to 428 and, optionally, the one or more processors initiate an action to generate alert(s) and/or modify an operational mode of at least one LIMD 102 to change the operational mode, similar to the actions and alerts discussed above at 322. Alert(s) can be saved in the memory of the external device, manually generated by a clinician, transmitted to designate entities, saved in a patient record, etc.

At 430, the one or more processors can display the diagnostics associated with the trended data to the clinician, such as in a chart, histogram, table, list, etc., as discussed above at 326.

At 432, the one or more processors can provide recommendations regarding the patient's care as discussed above at 328. Some recommendations may be provided in alerts as discussed above at 322.

Figure 6 illustrates a computer-implemented and/or processor-implemented method 600 that monitors AV conduction in a patient and controls pacing therapy of the patient having two LIMDs 102, 104. In some cases, the LIMD 102 is implanted in or on an atrial chamber, such as the RA, while the LIMD 104 is implanted in or on a ventricle, such as the RV. In some cases, the LIMDs 102, 104 are not communicating with each other, which saves power, unless the patient's heart rhythm requires the i2i communication for therapeutic reasons. In Figure 6, the LIMD 102, LIMD 104 can periodically and temporarily enable i2i communication and transition to a dual-chamber mode (e.g., DDD, etc.) to collect surrogate AV intervals. The diagnostic data can be trended by the LIMD 102 or LIMD 104. An external device 109 can communicate with one or both of the LIMDs 102, 104, receive the AV intervals, the diagnostic data, originally acquired CA data, marker data, etc., and display the trended data. In response to an evaluation of the diagnostic data satisfying one or more criteria, an alert can be generated.

The operations of Figure 6 may be implemented by hardware, firmware, circuitry and/or one or more processors housed partially and/or entirely within an LIMD 102, 104, an external device 109, wearable device, a local external device, remote server or more generally within a health care system. Optionally, the operations of Figure 6 may be shared across devices, and thus partially implemented by one or more of an LIMD 102, 104, an external device 109, wearable device, a local external device, remote server or more generally within a health care system. For example, the LIMDs 102, 104 include IMD memory and one or more IMD processors, the external device 109 includes external device memory and one or more external device processors, and further, other external devices/systems (e.g., local, remote or anywhere within the health care system) that may implement the operations of Figure 6 include external device memory and one or more external device processors.

Although the acquisition and processing of Figure 6 is written primarily from the viewpoint of the LIMD 102 implanted within, in or on an atrial chamber (e.g., RA), it should be understood that after timing data and/or CA signal data is acquired, the signals/data can be transmitted to another external device 109, another LIMD 104, or a different implanted device for evaluation/processing/storage. In other embodiments, the method of Figure 6 can primarily be accomplished by the LIMD 104 implanted within, on, or in a ventricular chamber (e.g., RV).

At 602, one or more processors initiate monitoring of AV conduction within a heart of a patient. In some embodiments, the one or more processor can select electrodes of the LIMD 102 and/or LIMD 104 for sensing. The surrogate AV interval can be measured at predetermined intervals or monitoring frequency, such as hourly, daily, weekly, monthly, etc. Optionally, the measurements can be generated when a patient is at rest or active, based on a clock time, detected sleep state, activity of the patient, posture of the patient (prone, sitting, standing, etc.). In still further embodiments, the measurements can be generated on demand, such as via a command received from an external device 109. In some embodiments, either the LIMD 102 or the LIMD 104 can be designated as a primary and initiate the monitoring.

At 604, the one or more processors initiate i2i communication between LIMD 102 and LIMD 104. In some embodiments, the LIMD 102 can initiate the i2i communication. When i2i communication is active and both LIMDs 102, 104 are active and communicating with each other, timing information is transmitted between the two devices. The timing information can include, for example, ventricular sense (VS) events, ventricular pace (VP) events, atrial sense (AS) events, and atrial pace (AP) events.

At 606, the one or more processors transition the LIMD 102 and the LIMD 104 to a dual-chamber operational mode, such as DDD, DDI, etc. In some embodiments, the transition to a dual-chamber operational mode 606 and the initiation of i2i communication 604 can occur simultaneously. In other embodiments, the transition to a dual-chamber operational mode at 606 may precede the initiation of i2i communication at 604.

At 608, the one or more processors initialize a detection period over which to sense the CA signals for a series of heartbeats. The detection period can be a predetermined time period, such as 10 seconds, 30 seconds, one minute, two minutes, etc. Alternatively, the detection period can be based on a predetermined number of heartbeats or a predetermined number of atrial events.

At 610, the one or more processors, such as of the LIMD 102, determine an atrial event, such as a first atrial event. The atrial events can be atrial pace (AP) events and/or atrial sense (AS) events. The one or more processors continue to monitor to determine the next or second atrial event.

At 612, the one or more processors monitor for an i2i message from the LIMD 104 that sends notification of a ventricular event (e.g., VS, VP).

At 614, the one or more processors determine whether the ventricular event is received before the next atrial event is determined or before a timeout window expires. In some cases, the timeout window can be set, such as by the physician, and may be based on the patient's previously determined surrogate AV interval, measured intrinsic AV interval, and/or determined A-A interval (e.g., averaged over a predetermined time). If the ventricular event is received, flow passes to 616. If no ventricular event is received before the timeout window expires or a next atrial event is determined, flow passes to 620.

At 616, the one or more processors determine a COI associated with the received ventricular event. For example, the COI can be a peak or start of the identified R-wave, or the VS event 514 (Figure 5) or a VP event, or other COI along the QRS complex. It should be understood that other COIs may be determined.

At 618, the one or more processors determine a time interval between the atrial event (e.g., the first atrial event (e.g., AP, AS) determined at 610) and the COI determined at 616. In this example the time interval is the surrogate AV interval (e.g., surrogate measurement of intrinsic AV delay). In Figure 5, AS and VP events are not shown, but the determination of time intervals equally applies for these events. As examples, time interval 512c is determined between AP event 504a and VS event 514c, and time interval 512d is determined between AP event 504b and VS event 514e. Not all of the time intervals 512 are indicated.

At 620, the one or more processors generate diagnostic data by updating trending statistics associated with the surrogate AV interval. If a ventricular event is not received at 614 before the next atrial event or the timeout expires, no surrogate AV interval is determined for that atrial event. The diagnostic data and trending statistics are discussed above at 316, 354, and 424. As the surrogate AV intervals data for the patient is acquired over time (e.g., multiple days, quarters, years), increasing amounts of data can be trended to help inform practitioners of a patient's progression of AV delay and/or AV conduction disease.

At 622, the one or more processors determine whether the detection period initialized at 608 is complete. If no, flow returns to 610 to determine the next atrial event. If the detection period is complete, flow passes to 624. It should be understood that the trending statistics can be updated at 620 to reflect data associated with all of the time intervals after the detection period of 608 is complete.

At 624, the one or more processors restore the previous operational mode in the atrial LIMD 102 and/or ventricular LIMD 104 and deactivate i2i communication (e.g., transition the LIMD 102, 104). Battery power within the LIMDs 102, 104 can be conserved by only utilizing the dual-chamber mode and the i2i communication when needed.

At 626, the one or more processors determine whether the trending statistics satisfy one or more criteria, such as one or more threshold, indicative of an increase in AV delay and/or progression of AV conduction disease. For example, the one or more processors can evaluate the diagnostic data generated at 620 against one or more criteria stored in the LIMD 102, 204 or the external device 109. In some embodiments, the one or more processors can evaluate the diagnostic data by applying a machine learning model trained to classify progression of AV conduction disease.

If the trending statistic(s) satisfy one or more criteria at 626, flow passes to 628 and, optionally, the one or more processors initiate an action to generate alert(s) and/or modify an operational mode of at least one LIMD 102, 104 to change the operational mode. For example, the criteria can be associated with operational mode(s). The diagnostic data and evaluation of the diagnostic data against one or more criteria stored in at least one of the LIMD 102, 104, and external device 109 can be used directly by the atrial LP to independently (e.g., without direct clinician involvement) assess over time any potential deterioration of intrinsic AV conduction performance and automatically transition to a dual-chamber pacing mode (e.g., DDD) when warranted (e.g., based on criteria configured by the clinician) to provide pacemaker-mediated therapeutic AV synchronization (e.g., tracking), and then transition back to non-dual chamber pacing mode (e.g., AAI, AAI+VVI) when durable intrinsic AV conduction is reestablished (e.g., based on additional criteria configured by the clinician). An example of mode switching is disclosed in U.S. pub. App. 2025/0108221, titled "Dual chamber leadless pacemaker systems and methods for use therewith", published April 3, 2025.

For example, the operational modes can be programmed/transitioned to provide dual-chamber pacing mode or non-dual chamber pacing mode. For example, if the operational modes of the LIMD 102 and LIMD 104 are sensing, the operational mode may be changed to pacing in the atria and sensing in both chambers. In another embodiment, the operational mode may be changed to pace in both chambers (e.g., DDD). In still further embodiments, the operational mode may be changed to DDI or VDD.

Accordingly, the LIMD(s) delivers a particular treatment for the medical condition of progression of AV conduction disease and/or heart block. The treatment is selected from the LIMDs' operational modes, and the treatment is selected based on the patient's particular stage of disease. Further, the battery use of the LIMD(s) is minimized if the patient does not require continuous or nearcontinuous pacing and/or i2i communication, resulting in a longer device life. In some embodiments, the change of the operational mode from a lower level of treatment (e.g., sensing) to a higher level of treatment (e.g., pacing) and back to a lower level of treatment may occur if the patient's surrogate AV interval is proximate a threshold, such that sometimes the diagnostic data are above the threshold and sometimes below the threshold. Thus, the patient is advantageously treated with the level of treatment needed, preventing both the overtreatment and undertreatment of the patient. In some cases, to prevent cycling back and forth between levels of treatment when the surrogate AV interval is proximate a threshold, one operational mode may be maintained for a predetermined period of time before the next change. In some other cases, hysteresis can be included with a threshold, wherein the effective threshold associated with positive changes of the patient's surrogate AV interval is relatively higher than the effective threshold associated with negative changes of the patient's surrogate AV interval.

Alert(s) can be saved in the memory of the LIMD 102. In some cases, the diagnostic data, time intervals, CA signals, atrial events, COIs, etc., can also be saved. Optionally, if more than one threshold is used, alerts may be associated with a particular threshold to provide a more detailed diagnostic to the clinician. In some embodiments, an alert can indicate that the patient's condition is deteriorating and should be more frequently monitored. In still further embodiments, an alert can include at least one recommendation to i) increase or decrease a monitoring frequency, ii) program an operational mode of one or more LIMD implanted in the heart, iii) increase or decrease the threshold, iv) add, modify, and/or remove an alert, vi) add, modify, and/or discontinue a medication, vii) add, modify, and/or remove a recommendation to the patient, and vii) change a pacing setting.

At 630, optionally, the one or more processors can transmit at least one of the diagnostic data (e.g., trended data), surrogate AV intervals, CA signals, the COIs, the atrial events, action(s) and alert(s) to an external device 109 and/or another implantable device (e.g., an LIMD, cardiac monitor). For example, wireless transceivers, communication coils and antenna, and/or conductive communication can be used to transmit data to an external device or other implanted device. In some embodiments, the transmission is initiated by the external device or other implanted device. In other embodiments, the LIMD 102 can be directed to wirelessly transmit an alert and/or other data to an external device 109.

In some cases, the data may be transmitted to a patient's local device, such as their phone or other device located near their person, and then further transmitted to other external devices, such as those located in a clinician's office, on a network, etc.

At 632, the one or more processors can display the diagnostics associated with the trended data to the clinician, such as in a chart, histogram, table, list, etc., as discussed at 326. As the surrogate AV intervals are acquired over time, increasing amounts of data can be trended to help inform practitioners of a patient's progression of AV delay and/or AV conduction disease. Alerts can be manually generated by a clinician, transmitted to designated entities, saved in a patient record, etc.

At 634, the one or more processors can provide recommendations regarding the patient's care, as discussed at 322. Some recommendations may be provided in alerts as discussed at 628. Recommendations can include, but are not limited to, i) increase or decrease a monitoring frequency, ii) program an operational mode of one or more LIMD 102, 104 implanted in the heart (e.g., change (e.g., reprogram) the operational mode of the LIMD 102 implanted in the RA and/or change (e.g., reprogram) the operational mode of the LIMD 104 implanted in the RV), iii) increase or decrease the threshold(s), iv) add, modify, and/or remove an alert, v) add, modify, and/or discontinue a medication, vi) add, modify, and/or remove a recommendation to the patient (e.g., lifestyle, activity level), and vii) change a pacing setting. Recommendations can be displayed on a monitor, printed, added to a patient file, texted, emailed, etc. It should be understood that some recommendations may be associated with alerts, not associated with alerts, generated by AI based on patient and/or collective data, etc.

In some embodiments, the method of Figure 6 can be initiated by using an external device 109. For example, the external device 109 can communicate with one or both of the LIMDs 102, 104, initiate i2i communication, and change the operational mode of one or both of the LIMDs 102, 104 to a dual-chamber mode. One or both of the LIMDs 102, 104 and the external device 109 can be capable of determining the atrial events, determining the COI associated with ventricular events, and processing the data. The external device 109 can display data and recommendations (e.g., increase/decrease monitoring, no action, implant LIMD with different capabilities, change operational mode). Data and recommendations can be transmitted to designated accounts, alerts provided, etc. The external device 109 can restore the operational mode of one or both of the LIMDs 102, 104 to the previous mode, or retain the operational mode of one or both of the LIMDs 102, 104, maintaining i2i communication and/or the dual-chamber operational mode.

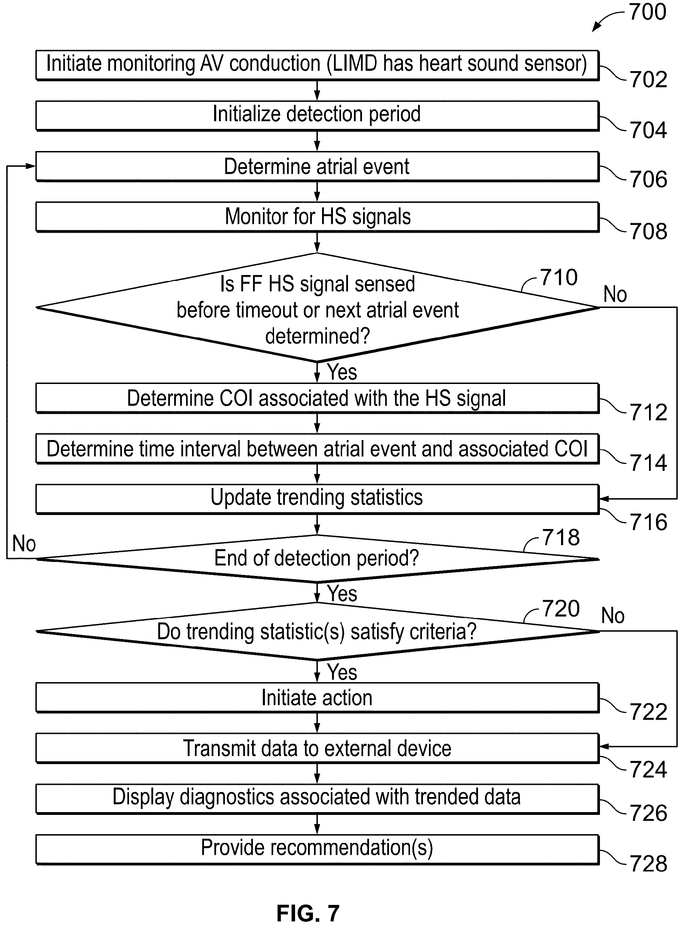

Figure 7 illustrates a computer-implemented and/or processor-implemented method 700 that, using an LIMD and an implanted HS sensor, monitors AV conduction in a patient and controls pacing therapy of the patient. The method can utilize an LIMD 102 implanted in, within, or on a chamber of the heart to sense CA signals of the local chamber (e.g., NF CA signals). In some embodiments, the LIMD 102 can be implanted in the RA. The LIMD 102 includes an HS sensor, such as accelerometer 154 in Figure 1B and monitoring sensor 188 in Figure 1C, for detecting HS signals that that are associated with cardiac valve closures, indicating such as heart sounds S1, S2, S3, S4. Time intervals can be determined between atrial events associated with the local chamber and COIs associated with the HS signals, and trending statistics can be updated. In response to the trending statistics indicating an increase in the surrogate AV intervals or increase in AV conduction disease, an alert can be generated or an operational mode of an implanted LIMD 102 can be changed, among other actions discussed below.

The operations of Figure 7 may be implemented by hardware, firmware, circuitry and/or one or more processors housed partially and/or entirely within an LIMD 102, an external device 109, wearable device, a local external device, remote server or more generally within a health care system. Optionally, the operations of Figure 7 may be shared across devices, and thus partially implemented by one or more of an LIMD 102, an external device 109, wearable device, a local external device, remote server or more generally within a health care system. For example, the LIMD 102 includes IMD memory and one or more IMD processors, the external device 109 includes external device memory and one or more external device processors, and further, other external devices/systems (e.g., local, remote or anywhere within the health care system) that may implement the operations of Figure 7 include external device memory and one or more external device processors.

Although the method of Figure 7 is written primarily from the viewpoint of the LIMD 102 implanted in the RA, it should be understood that after signal data, such as the NF CA signals and HS signals, as well as atrial events, are sensed, determined, or acquired, the data can be transmitted to an external device or a different implanted device for evaluation/processing/storage.

At 702, one or more processors initiate monitoring of AV conduction within a heart of a patient and optionally select electrodes of the LIMD 102 for sensing. In some embodiments, a single LIMD 102 including a HS sensor can be implanted in or on the RA, which can be referred to as the local chamber. The surrogate AV interval can be measured at predetermined intervals or monitoring frequency, such as hourly, daily, weekly, monthly, etc. Optionally, the measurements can be generated when a patient is at rest or active, based on a clock time, detected sleep state, activity of the patient, posture of the patient (prone, sitting, standing, etc.). In still further embodiments, the measurements can be generated on demand, such as via a command received from an external device 109.

At 704, the one or more processors initialize a detection period over which to simultaneously sense NF CA signals using, for example, electrodes of the LIMD 102, and detect HS signals. The HS signals can also be referred to as seismocardiogram (SCG) signals and are detected by a HS sensor such as an accelerometer 154 in Figure 1B and monitoring sensor 188 in Figure 1C. The CA signals and HS signals are sensed over a predetermined time period, such as 10 seconds, 30 seconds, one minute, two minutes, etc. In other embodiments, the detection period can be based on a predetermined or minimum number of heartbeats, such as 10 heartbeats, 30 heartbeats, 60 heartbeats, etc., or a predetermined number of atrial events. The HS signals can include indications associated with S1, S2, S3, S4, etc.

At 706, the one or more processors determine an atrial event, such as a first atrial event. The atrial event can be an atrial pace (AP) event and/or an atrial sense (AS) event. The one or more processors continue to monitor to determine the next or second atrial event.

At 708, the one or more processors monitor, using the HS sensor of the LIMD 102, for the HS signals that include at least one heart sound (e.g., S1).

At 710, the one or more processors determine whether the HS signal is sensed before the next atrial event is determined or before a timeout window expires. In some cases, the timeout window can be set, such as by the physician, and may be based on the patient's previously determined surrogate AV interval, measured intrinsic AV interval, and/or determined A-A interval (e.g., averaged over a predetermined time). If the HS signal is sensed, flow passes to 712. If no HS signal is sensed before the timeout window expires or a next atrial event is determined, flow passes to 716.

At 712, the one or more processors determine a COI associated with the HS signal. For example, the COI can be the S1 heart sound. It should be understood that other COIs may be determined.

At 714, the one or more processors determine a time interval between the atrial event (e.g., the first atrial event (e.g., AP, AS) determined at 706) and the COI determined at 712. The time interval can be a surrogate AV interval (e.g., surrogate measurement of intrinsic AV delay). If a HS signal is not identified at 710 before the next atrial event, no time interval is determined for that atrial event.

At 716, the one or more processors generate diagnostic data by updating trending statistics associated with the surrogate AV interval, as discussed above at 316.

At 718, the one or more processors determine whether the detection period initialized at 704 is complete. If no, flow returns to 706 to determine the next atrial event. If the detection period is complete, flow passes to 720. It should be understood that the trending statistics can be updated at 716 to reflect data associated with all of the time intervals after the detection period of 718 is complete.

In other embodiments, the detection of NF CA signals and HS signals can be ongoing, as discussed in Figure 3B, such as to continue until a command to stop is received, such as from an external device. The collection of CA and HS signals over an extended period of time can provide the advantage of insights that can be correlated to sleep, motion/activity, posture, as well as monitoring for an accelerated increase in the surrogate AV intervals.

At 720, the one or more processors determine whether the trending statistics satisfy one or more criteria, such as one or more threshold, indicative of an increase in AV delay and/or progression of AV conduction disease as discussed above at 320. For example, the one or more processors can evaluate the diagnostic data generated at 716 against one or more criteria stored in the LIMD 102 or the external device 109. In some embodiments, the one or more processors can evaluate the diagnostic data by applying a machine learning model trained to classify progression of AV conduction disease.

If the trending statistic(s) satisfy one or more criteria at 720, flow passes to 722 and, optionally, as discussed above at 322, the one or more processors initiate an action to generate alert(s) and/or modify an operational mode of at least one LIMD 102 to change the operational mode. Alert(s) can be saved in the memory of the LIMD 102. In some cases, the diagnostic data, time intervals, CA signals, HS signals, atrial events, COIs, etc., can also be saved. In some embodiments, the one or more processors determine whether one or more of the diagnostic data satisfy one or more threshold indicative of increased surrogate AV intervals and/or progression of AV conduction disease. For example, a threshold of 200 milliseconds (ms), 280 ms, 300 ms, etc., can be determined. In other embodiments, one or more threshold can be programmable by the clinician and can be based on a particular patient, a population of patients, and the like. In still other embodiments, the one or more criteria may include a threshold for percentage of cycles without FF HS detection. This threshold may also be programmable by a clinician, and may provide, for example, an indicator of a patient's progression to second- or third-degree heart block. In some embodiments, a threshold of 10%, 20%, 30%, etc., may be selected. In general, the one or more criteria may include one or more thresholds associated with one or more of the trending statistics.

At 724, optionally, the one or more processors can transmit at least one of the diagnostic data (e.g., trended data), surrogate AV intervals, CA signals, HS signals, the COIs, the atrial events, and the alert(s) to an external device 109 and/or another implantable device (e.g., an LIMD 104, cardiac monitor). For example, wireless transceivers, communication coils and antenna, and/or conductive communication can be used to transmit data to an external device or other implanted device. In some embodiments, the transmission is initiated by the external device or other implanted device. In other embodiments, the LIMD 102 can be directed to wirelessly transmit an alert and/or other data to an external device 109.

At 726, the one or more processors can display the diagnostics associated with the trended data to the clinician, such as in a chart, histogram, table, list, etc., as discussed at 326. As the surrogate AV intervals are acquired over time, increasing amounts of data can be trended to help inform practitioners of a patient's progression of AV delay and/or AV conduction disease. Alerts can be manually generated by a clinician, transmitted to designated entities, saved in a patient record, etc.

At 728, the one or more processors can provide recommendations regarding the patient's care, as discussed at 328. Some recommendations may be provided in alerts as discussed at 322. Recommendations can be displayed on a monitor, printed, added to a patient file, texted, emailed, etc.

In some embodiments, the HS sensor can be incorporated into a different LIMD, such as an LIMD 104 implanted in a ventricle, such as the RV. In this example, one of the LIMDs 102, 104 can initiate i2i communication and the LIMD 104 can transmit the HS signal data and/or associated COIs to the LIMD 102. In other embodiments, the LIMD 104 can transmit the HS signal data and/or associated COIs to the external device. In still further embodiments, the HS sensor can be incorporated into a different device, such as a different implantable device (e.g., ICM), an external device such as a handheld device, wearable, Holter monitor, application via a smartphone, etc. The HS signal data can be transmitted to the LIMD 102 and/or external device 109.

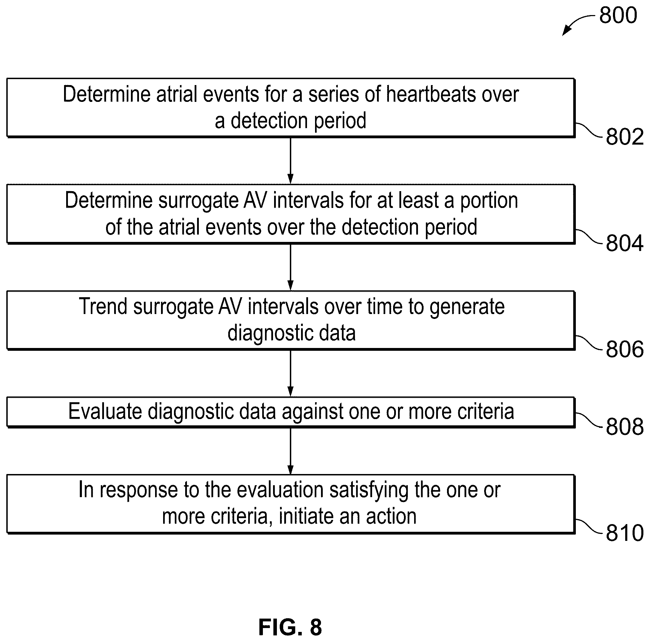

Figure 8 illustrates still another computer-implemented and/or processor-implemented method 800 that monitors AV conduction in a patient and controls pacing therapy of the patient. The operations of Figure 8 may be implemented by hardware, firmware, circuitry and/or one or more processors housed partially and/or entirely within an LIMD 102, an external device 109, wearable device, a local external device, remote server or more generally within a health care system, as discussed above with respect to Figures 3A, 3B, 4, 6, and 7.

At 802, one or more processors determine atrial events for a series of heartbeats over a detection period using a leadless implantable medical device (LIMD) 102 implanted in or on an atrial chamber of a heart.

At 804, the one or more processors determine surrogate AV intervals for at least a portion of the atrial events over the detection period based on a signal indicative of ventricular activity.

At 806, the one or more processors trend the surrogate AV intervals over time to generate diagnostic data.

At 808, the one or more processors evaluate the diagnostic data against one or more criteria stored in at least one of the LIMD 102 or an external device 109.

At 810, the one or more processors, in response to the evaluation satisfying the one or more criteria, initiate an action to modify an operational mode of at least one LIMD 102, 104 or to generate an alert, the action comprising at least one of: changing the operational mode of at least one LIMD 102, 104 or transmitting the alert to the external device 109.

In some embodiments, the one or more processors determine the surrogate AV intervals by sensing, with electrodes of the LIMD 102, a far-field (FF) cardiac activity signal associated with a ventricular chamber, the FF cardiac activity signal including the signal indicative of ventricular activity. In other embodiments, the one or more processors determine the surrogate AV intervals by detecting a heart sound signal using a heart sound (HS) sensor disposed in the LIMD 102, the heart sound signal including the signal indicative of ventricular activity. In further embodiments, the one or more processors determine the atrial events over a second detection period, determine the surrogate AV intervals over the second detection period, and update the trending of the surrogate AV intervals over time to generate updated diagnostic data. In some embodiments, the one or more processors transmit the diagnostic data or the surrogate AV intervals to the external device.

In other embodiments, the one or more processors transition the LIMD 102 from a non-dual chamber operating mode to a dual-chamber operating mode, receive the signal indicative of ventricular activity from a second LIMD 104, and restore a previous operational mode of the LIMD 102 at an expiration of the detection period. In some cases, in advance of, concurrent with, or after programming the LIMD 102, the one or more processors enable implant-to-implant (i2i) communication between the LIMD 102 and the second LIMD 104, and in response to restoring the previous operation mode, the one or more processors deactivate the i2i communication.

In still further embodiments, the action comprises generating the alert to prompt at least one of: reprogramming the LIMD 102, implanting a second LIMD 104 in a ventricular chamber, or reprogramming a second LIMD 104 implanted in a ventricular chamber. In further cases, evaluating the diagnostic data includes the one or more processors applying a machine learning model trained to classify progression of AV conduction disease. In other embodiments, the one or more processors determine that no surrogate AV interval associated with a first atrial event can be determined when the signal indicative of ventricular activity is not detected within a predetermined timeout window following the first atrial event or when a second atrial event is detected before i) the signal indicative of ventricular activity is detected or ii) the predetermined timeout window expires.

Applicable to all methods disclosed herein, in some embodiments, all processing of signals and trending statistics can be accomplished by a single LIMD 102, 104. In other embodiments, processing of signals and trending statistics can be partially accomplished by a single LIMD 102, 104 and partially accomplished by an external device 109. In still further embodiments, processing of signals and trending statistics can be accomplished by the external device 109. In other embodiments, processing of signals and trending statistics can be partially accomplished by a first LIMD 102 and partially accomplished by a second LIMD 104. In other cases, processing of signals and trending statistics can be partially accomplished by a first LIMD 102, partially accomplished by a second LIMD 104, and partially accomplished by the external device 109.

An aspect of the invention relates toa system 100 for monitoring AV conduction and controlling pacing therapy. The system 100 comprising a LIMD 102 configured to be implanted in or on an atrial chamber of a heart 101. The system 100 also comprising a memory 184 storing program instructions, and one or more processors 182 that, when executing the program instructions, are configured to utilize the program instructions to determine atrial events for a series of heartbeats over a detection period using the LIMD 102. The one or more processors 182 are also configured to determine surrogate AV intervals for at least a portion of the atrial events over the detection period based on a signal indicative of ventricular activity. The one or more processors 182 are further configured to trend the surrogate AV intervals over time to generate diagnostic data, and evaluate the diagnostic data against one or more criteria stored in at least one of the LIMD 102) or an external device 109. The one or more processors 182 are additionally configured to, in response to the evaluation satisfying the one or more criteria, initiate an action to modify an operational mode of at least one LIMD 102, 104 or to generate an alert. The action comprises at least one of: change the operational mode or transmit the alert to the external device 109.

In an embodiment, the LIMD 102 further comprises electrodes 172, 174 configured to sense a FF cardiac activity signal associated with a ventricular chamber. The FF cardiac activity signal includes the signal indicative of ventricular activity.

In an embodiment, the electrodes further comprise at least three electrodes 234, 240, 242. In this embodiment, the one or more processors 182 are further configured to select two of the at least three electrodes 234, 240, 242 to maximize the FF cardiac activity signal relative to the atrial events.

In an embodiment, the LIMD 102 further comprises a heart sound (HS) sensor 154, 188 configured to detect a heart sound signal that comprises an S1 heart sound. In this embodiment, the signal indicative of ventricular activity is based on the heart sound signal.

In an embodiment, the one or more processors 182 are further configured to determine the atrial events over a second detection period, determine the surrogate AV intervals over the second period, and update the trending of the surrogate AV intervals over time to generate updated diagnostic data.

In an embodiment, the one or more processors 182 are further configured to transmit the diagnostic data or the surrogate AV intervals to the external device 109.

In an embodiment, the one or more processors 182 are further configured to transition the LIMD 102 from a non-dual chamber operating mode to a dual-chamber operating mode. The one or more processors 182 are also, in this embodiment, configured to receive the signal indicative of ventricular activity from a second LIMD 104, and restore a previous operational mode of the LIMD 102 at an expiration of the detection period.

In an embodiment, the one or more processors 182 are further configured to enable i2i communication between the LIMD 102 and the second LIMD 104, and in response to restoring the previous operation mode, deactivate the i2i communication.

In an embodiment, the one or more processors 182 are further configured to generate the alert to prompt at least one of: reprogramming the LIMD 102, implanting a second LIMD 104 in a ventricular chamber, or reprogramming a second LIMD 104 implanted in a ventricular chamber.

In an embodiment, the evaluate the diagnostic data comprises the one or more processors 182 applying a machine learning model trained to classify progression of AV conduction disease.

In an embodiment, the one or more processors 182 are further configured to determine that no surrogate AV interval associated with a first atrial event can be determined when the signal indicative of ventricular activity is not detected within a predetermined timeout window following the first atrial event or when a second atrial event is detected before i) the signal indicative of ventricular activity is detected or ii) the predetermined timeout window expires.

In an embodiment, the atrial events comprise at least one of atrial pace (AP) events or atrial sense (AS) events.

In an embodiment, the one or more criteria is programmable.

In an embodiment, the diagnostic data comprises at least one of i) average deviation AV interval, ii) standard deviation AV interval, iii) minimum and maximum AV interval, iv) average of the difference between AV intervals from consecutive cardiac cycles, v) standard deviation of the difference between AV intervals from consecutive cardiac cycles, vi) percentage of cycles without FF CA detection, vii) binned histograms of AV intervals, or viii) binned histograms of differences between AV intervals from consecutive cardiac cycles.

In an embodiment, the diagnostic data can be managed as moving-window statistics or exponentially filtered statistics.

Another aspect of the invention relates to a system 100 for monitoring atrioventricular (AV) delay within a heart 101. The system 100 comprising a LIMD 102 configured to be implanted in a local chamber of the heart 101. A local chamber is an atrium. The LIMD 102 comprising one or more electrodes 172, 174 configured to at least one of sense cardiac activity (CA) signals or deliver therapy. The system 100, such as the LIMD 102, also comprising memory 184 to store program instructions and one or more processors 182 that, when executing the program instructions, are configured to determine atrial events for a series of heartbeats over a time period. The one or more processors 182 are also configured to receive far-field (FF) signals indicative of FF activity of a remote chamber of the heart 101 for the series of heartbeats over the time period. The one or more processors 182 are further configured to identify a characteristic of interest (COI) associated with the FF signals, and determine time intervals between the atrial events and associated COIs. The one or more processors 182 are additionally configured to generate an alert in response to i) one of the time intervals or ii) a plurality of the time intervals satisfying a threshold indicative of an increase in AV delay.

In an embodiment, the FF signals comprise FF CA signals. In this embodiment, the remote chamber is a ventricle, and the one or more processors 182 are configured to receive the FF CA signals indicative of ventricular activity in the ventricle, the time intervals representing AV time intervals.

In an embodiment, the FF signals comprise FF heart sound (HS) signals. In this embodiment, the remote chamber is a ventricle, and the one or more processors 182 are configured to receive the FF HS signals indicative of ventricular activity in the ventricle, the time intervals representing AV time intervals.

In an embodiment, the one or more processors 182 are further configured to generate a second alert in response to i) a second one of the time intervals or ii) a second plurality of the time intervals satisfying a second threshold. In this embodiment, the second threshold is indicative of a second increase in the AV delay.

In an embodiment, the atrial events comprise at least one of atrial pace (AP) events or atrial sense (AS) events.

In an embodiment, the FF signals comprise FF CA signals. In this embodiment, the one or more electrodes comprises at least three electrodes 234, 240, 242, and the one or more processors 182 are further configured to select two of the at least three electrodes 234, 240, 242 that maximizes the FF CA signal relative to the NF CA signal.

In an embodiment, the LIMD 102 is further configured to wirelessly transmit at least one of the CA signals, the COI, the FF signals, the atrial events, the time intervals, or the alert to an external device 109.

In an embodiment, the external device 109 further comprises memory to store program instructions, and one or more processors that, when executing the program instructions, are configured to receive or collect an electrocardiogram (ECG) associated with the heart, determine a second COI associated with R-waves within the ECG, and determine second time intervals between the atrial events received from the LIMD 102 and associated second COIs.

In an embodiment, the the threshold is programmable.

In an embodiment, the alert comprises diagnostics associated with trending the time intervals.

A further aspect of the invention relates to a system 100 for monitoring AV delay within a heart 101. The system 100 comprising a first LIMD 102 configured to be implanted in an atrium of the heart 101. The first LIMD 102 comprising a first set of one or more electrodes 172, 174 to at least one of sense cardiac activity (CA) signals or deliver therapy. The system 100 also comprises a second LIMD 104 configured to be implanted in a ventricle of the heart 101. The second LIMD 104comprising a second set of one or more electrodes 172, 174 to at least one of sense CA signals or deliver therapy. The system 100 further comprises memory 184 to store program instructions, and one or more processors 182, that when executing the program instructions, are configured to activate implant-to-implant (i2i) communication between the first and second LIMDs 102, 104. The one or more processors 182 are also configured to determine atrial events for a series of heartbeats over a time period, and determine ventricular events for the series of heartbeats over the time period. The one or more processors 182 are further configured to determine time intervals between the atrial events and ventricular events for the series of heartbeats over the time period. The one or more processors 184 are additionally configured to generate an alert in response to one or more of the time intervals satisfying a threshold indicative of progression of the AV delay.

In an embodiment, the ventricular events comprise a ventricular sensed (VS) event or a ventricular pulsed (VP) event.

In an embodiment, the system 100 further comprising an external device 109. In this embodiment, the external device 109 if further configured to communicate with at least one of the first and second LIMDs 102, 104 to activate the i2i communication.

In an embodiment, the one or more processors 182 are further configured to program at least one of the first and second LIMDs 102, 104 to a dual chamber pacing mode.

In an embodiment, the one or more processors 182 are further configured to i) restore a previous operational mode in at least one of the first and second LIMDs 102, 104, or ii) deactivate i2i communication between the first and second LIMDs 102, 104.

In an embodiment, in response to the time intervals not satisfying the threshold, the one or more processors 182 are further configured to restore a previous operational mode in at least one of the first and second LIMDs 102, 104.

In an embodiment, in response to the time intervals satisfying the threshold, the one or more processors 182 are further configured to retain the dual chamber pacing mode in at least one of the first and second LIMDs 102, 104.

Yet another aspect of the invention relates to a system 100 for monitoring AV delay within a heart 101. The system 100 comprising a LIMD 102 configured to be implanted in an atrium of the heart 101. The LIMD 102 comprising one or more electrodes 172, 174 to at least one of sense cardiac activity (CA) signals or deliver therapy. The system 100, such as the LIMD 102, also comprising a memory 184 to store program instructions, and one or more processors 182 that, when executing the program instructions, are configured to determine atrial events for a series of heartbeats over a time period. The system 100 further comprising an external device 100 comprising external device memory to store external device program instructions, and one or more external device processors that, when executing the external device program instructions, are configured to collect an electrocardiogram (ECG) associated with the heart. The one or more external device processors are also configured to, receive the atrial events from the LIMD 102, and determine a characteristic of interest (COI) associated with R-waves within the ECG. The one or more external device processors are further configured to determine time intervals between the atrial events received from the LIMD 102 and associated COIs; and display diagnostics associated with trending the timing intervals.

In an embodiment, in response to one or more of the time intervals satisfying a threshold indicative of increased AV delay, the one or more external device processors are further configured to generate an alert.

In an embodiment, the threshold is programmable.

Embodiments may be implemented in connection with one or more IMDs. Non-limiting examples of IMDs include one or more of neurostimulator devices, implantable leadless monitoring and/or therapy devices, and/or alternative implantable medical devices. For example, the IMD may represent a cardiac monitoring device, pacemaker, cardioverter, cardiac rhythm management device, defibrillator, neurostimulator, leadless monitoring device, leadless pacemaker, and the like. The IMD may measure electrical and/or mechanical information. For example, the IMD may include one or more structural and/or functional aspects of the device(s) described in U.S. Pub. No. 2015/0335894, titled "Communications Scheme for Distributed Leadless Implantable Medical Devices Enabling Mode Switching", filed May 20, 2014; EP 3843 828 B1, titled "Adaptive VFA Cardiac Therapy", filed August 30, 2019; U.S. Patent Number 9,333,351, titled NEUROSTIMULATION METHOD AND SYSTEM TO TREAT APNEA, and U.S. Patent Number 9,044,610, titled SYSTEM AND METHODS FOR PROVIDING A DISTRIBUTED VIRTUAL STIMULATION CATHODE FOR USE WITH AN IMPLANTABLE NEUROSTIMULATION SYSTEM issued June 02, 2015. The IMD may monitor transthoracic impedance, such as implemented by the CorVue algorithm offered by St. Jude Medical. Additionally or alternatively, the IMD may include one or more structural and/or functional aspects of the device(s) described in U.S. Patent Number 9,216,285, titled LEADLESS IMPLANTABLE MEDICAL DEVICE HAVING REMOVABLE AND FIXED COMPONENTS issued December 22, 2015, and U.S. Patent Number 8,831,747, titled LEADLESS NEUROSTIMULATION DEVICE AND METHOD INCLUDING THE SAME issued September 09, 2014. Additionally or alternatively, the IMD may include one or more structural and/or functional aspects of the device(s) described in U.S. Patent Number 8,391,980, titled METHOD AND SYSTEM FOR IDENTIFYING A POTENTIAL LEAD FAILURE IN AN IMPLANTABLE MEDICAL DEVICE issued March 05, 2013, and U.S. Patent Number 9,232,485, titled SYSTEM AND METHOD FOR SELECTIVELY COMMUNICATING WITH AN IMPLANTABLE MEDICAL DEVICE issued January 05, 2016. Additionally or alternatively, the IMD may be a subcutaneous IMD that includes one or more structural and/or functional aspects of the device(s) described in U.S. Patent Number 10,765,860, titled SUBCUTANEOUS IMPLANTATION MEDICAL DEVICE WITH MULTIPLE PARASTERNAL-ANTERIOR ELECTRODES filed May 07, 2018; U.S. Patent Number 10,722,704, titled IMPLANTABLE MEDICAL SYSTEMS AND METHODS INCLUDING PULSE GENERATORS AND LEADS filed May 07, 2018; U.S. Patent Number 11,045,643, titled SINGLE SITE IMPLANTATION METHODS FOR MEDICAL DEVICES HAVING MULTIPLE LEADS, filed May 07, 2018. Additionally or alternatively, the IMD and related sensors, devices, systems, etc., as described herein can be at least one of the IMDs, sensors, devices, systems, etc., illustrated and described in U.S. Pub. No. 2022/0361837, filed February 8, 2022, titled METHOD AND SYSTEM FOR MONITORING HEART FUNCTION BASED ON HEART SOUND CENTER OF MASS; U.S. Pub. No. 2022/0361818, filed February 8, 2022, titled METHOD AND SYSTEM FOR MONITORING HEART FUNCTION BASED ON HEART SOUND CENTER OF MASS; U.S. Serial No. 18/743,533, filed June 14, 2024, titled METHOD AND SYSTEM FOR MONITORING HEART FUNCTION BASED ON ELECTROMECHANICAL ACTIVATION TIME; U.S. Serial No. 63/685,818, filed August 22, 2024, titled METHODS AND SYSTEMS FOR USING SEISMOCARDIOGRAPHY-BASED ALGORITHM FOR MONITORING HEART VALVE OPERATION, and U.S. Patent Number 11,071,872, filed January 7, 2019, titled Systems and methods for performing pacing using multiple leadless pacemakers. Further, one or more combinations of IMDs may be utilized from the above mentioned patents and applications in accordance with embodiments herein. Embodiments may be implemented in connection with one or more subcutaneous implantable medical devices (S-IMDs). For example, the S-IMD may include one or more structural and/or functional aspects of the device(s) described in U.S. Patent Number 10,722,704, titled IMPLANTABLE MEDICAL SYSTEMS AND METHODS INCLUDING PULSE GENERATORS AND LEADS, filed May 07, 2018; U.S. Patent Number 10,765,860, titled SUBCUTANEOUS IMPLANTATION MEDICAL DEVICE WITH MULTIPLE PARASTERNAL-ANTERIOR ELECTRODES, filed May 07, 2018. The IMD may represent a passive device that utilizes an external power source and/or an active device that includes an internal power source. The IMD may deliver some type of therapy/treatment, provide mechanical circulatory support and/or merely monitor one or more physiologic characteristics of interest (e.g., PAP, CA signals, impedance, heart sounds). Additionally or alternatively, embodiments may be implemented in connection with one or more passive IMDS (PIMDs). Non-limiting examples of PIMDs may include passive wireless sensors used by themselves or incorporated into or used in conjunction with other IMDs, such as cardiac monitoring devices, pacemakers, cardioverters, cardiac rhythm management devices, defibrillators, neurostimulators, leadless monitoring devices, leadless pacemakers, replacement valves, shunts, grafts, drug elution devices, blood glucose monitoring systems, orthopedic implants, and the like. For example, embodiments may implement one or more structural and/or functional aspects of the device(s) described in U.S. Patent No. 9,265,428 titled IMPLANTABLE WIRELESS SENSOR, U.S. Patent No. 8,278,941 titled STRAIN MONITORING SYSTEM AND APPARATUS, U.S. Patent No. 8,026,729 titled SYSTEM AND APPARATUS FOR IN-VIVO ASSESSMENT OF RELATIVE POSITION OF AN IMPLANT, U.S. Patent No. 8,870,787 titled VENTRICULAR SHUNT SYSTEM AND METHOD, and U.S. Patent No. 9,653,926 titled PHYSICAL PROPERTY SENSOR AND ACTIVE ELECTRONIC CIRCUIT AND WIERELESS POWER AND DATA TRANSMISSION.
Additionally or alternatively, embodiments herein may be implemented in connection with the methods and systems described in U.S. Pub. No. 2021/0345891, filed on May 08, 2020, titled METHOD AND DEVICE FOR DETECTING RESPIRATION ANOMALY FROM LOW FREQUENCY COMPONENT OF ELECTRICAL CARDIAC ACTIVITY SIGNALS; Patent Number 11,980,472, filed March 5, 2021, titled SYSTEM FOR VERIFYING A PATHOLOGIC EPISODE USING AN ACCELEROMETER; and U.S. Pub. No. 2023/0414951, filed June 19, 2023, titled SYSTEM AND METHOD FOR IMPLANTABLE MEDICAL DEVICE REMOTE PROGRAMMING

### Closing Statements

It should be clearly understood that the various arrangements and processes broadly described and illustrated with respect to the Figures, and/or one or more individual components or elements of such arrangements and/or one or more process operations associated of such processes, can be employed independently from or together with one or more other components, elements and/or process operations described and illustrated herein. Accordingly, while various arrangements and processes are broadly contemplated, described, and illustrated herein, it should be understood that they are provided merely in illustrative and non-restrictive fashion, and furthermore can be regarded as but mere examples of possible working environments in which one or more arrangements or processes may function or operate.

As will be appreciated by one skilled in the art, various aspects may be embodied as a system, method, or computer (device) program product. Accordingly, aspects may take the form of an entirely hardware embodiment or an embodiment including hardware and software that may all generally be referred to herein as a "circuit," "module" or "system." Furthermore, aspects may take the form of a computer (device) program product embodied in one or more computer (device) readable storage medium(s) having computer (device) readable program code embodied thereon.

Any combination of one or more non-signal computer (device) readable medium(s) may be utilized. The non-signal medium may be a storage medium. A storage medium may be, for example, an electronic, magnetic, optical, electromagnetic, infrared, or semiconductor system, apparatus, or device, or any suitable combination of the foregoing. More specific examples of a storage medium would include the following: a portable computer diskette, a hard disk, a random-access memory (RAM), a dynamic random-access memory (DRAM), a read-only memory (ROM), an erasable programmable read-only memory (EPROM or Flash memory), a portable compact disc read-only memory (CD-ROM), an optical storage device, a magnetic storage device, or any suitable combination of the foregoing.

Program code for carrying out operations may be written in any combination of one or more programming languages. The program code may execute entirely on a single device, partly on a single device, as a stand-alone software package, partly on single device and partly on another device, or entirely on the other device. In some cases, the devices may be connected through any type of network, including a local area network (LAN) or a wide area network (WAN), or the connection may be made through other devices (for example, through the Internet using an Internet Service Provider) or through a hard wire connection, such as over a USB connection. For example, a server having a first processor, a network interface, and a storage device for storing code may store the program code for carrying out the operations and provide this code through its network interface via a network to a second device having a second processor for execution of the code on the second device.

Aspects are described herein with reference to the Figures, which illustrate example methods, devices, and program products according to various example embodiments. These program instructions may be provided to a processor of a general-purpose computer, special purpose computer, or other programmable data processing device or information handling device to produce a machine, such that the instructions, which execute via a processor of the device implement the functions/acts specified. The program instructions may also be stored in a device readable medium that can direct a device to function in a particular manner, such that the instructions stored in the device readable medium produce an article of manufacture including instructions which implement the function/act specified. The program instructions may also be loaded onto a device to cause a series of operational steps to be performed on the device to produce a device implemented process such that the instructions which execute on the device provide processes for implementing the functions/acts specified.

The units/modules/applications herein may include any processorbased or microprocessor-based system including systems using microcontrollers, reduced instruction set computers (RISC), application specific integrated circuits (ASICs), field-programmable gate arrays (FPGAs), logic circuits, and any other circuit or processor capable of executing the functions described herein. Additionally or alternatively, the modules/controllers herein may represent circuit modules that may be implemented as hardware with associated instructions (for example, software stored on a tangible and non-transitory computer readable storage medium, such as a computer hard drive, ROM, RAM, or the like) that perform the operations described herein. The above examples are exemplary only and are thus not intended to limit in any way the definition and/or meaning of the term "controller." The units/modules/applications herein may execute a set of instructions that are stored in one or more storage elements, to process data. The storage elements may also store data or other information as desired or needed. The storage element may be in the form of an information source or a physical memory element within the modules/controllers herein. The set of instructions may include various commands that instruct the modules/applications herein to perform specific operations such as the methods and processes of the various embodiments of the subject matter described herein. The set of instructions may be in the form of a software program. The software may be in various forms such as system software or application software. Further, the software may be in the form of a collection of separate programs or modules, a program module within a larger program or a portion of a program module. The software also may include modular programming in the form of object-oriented programming. The processing of input data by the processing machine may be in response to user commands, or in response to results of previous processing, or in response to a request made by another processing machine.

It is to be understood that the subject matter described herein is not limited in its application to the details of construction and the arrangement of components set forth in the description herein or illustrated in the drawings hereof. The subject matter described herein is capable of other embodiments and of being practiced or of being carried out in various ways. Also, it is to be understood that the phraseology and terminology used herein is for the purpose of description and should not be regarded as limiting. The use of "including," "comprising," or "having" and variations thereof herein is meant to encompass the items listed thereafter and equivalents thereof as well as additional items.

It is to be understood that the above description is intended to be illustrative, and not restrictive. For example, the above-described embodiments (and/or aspects thereof) may be used in combination with each other. In addition, many modifications may be made to adapt a particular situation or material to the teachings herein without departing from its scope. While the dimensions, types of materials and coatings described herein are intended to define various parameters, they are by no means limiting and are illustrative in nature. Many other embodiments will be apparent to those of skill in the art upon reviewing the above description. The scope of the embodiments should, therefore, be determined with reference to the appended claims, along with the full scope of equivalents to which such claims are titled. In the appended claims, the terms "including" and "in which" are used as the plain-English equivalents of the respective terms "comprising" and "wherein." Moreover, in the following claims, the terms "first," "second," and "third," etc. are used merely as labels, and are not intended to impose numerical requirements on their objects or order of execution on their acts.

## Claims

1. A system (100) for monitoring atrioventricular, AV, conduction and controlling pacing therapy, the system (100) comprising:
a leadless implantable medical device, LIMD, (102) configured to be implanted in or on an atrial chamber of a heart (101);
a memory (184) storing program instructions; and
one or more processors (182) that, when executing the program instructions, are configured to utilize the program instructions to:
determine atrial events for a series of heartbeats over a detection period using the LIMD (102);
determine surrogate AV intervals for at least a portion of the atrial events over the detection period based on a signal indicative of ventricular activity;
trend the surrogate AV intervals over time to generate diagnostic data;
evaluate the diagnostic data against one or more criteria stored in at least one of the LIMD (102) or an external device (109); and
in response to the evaluation satisfying the one or more criteria, initiate an action to modify an operational mode of at least one LIMD (102, 104) or to generate an alert, wherein the action comprises at least one of: change the operational mode or transmit the alert to the external device (109).

2. The system of claim 1, wherein the LIMD (102) further comprises electrodes (172, 174) configured to sense a far-field (FF) cardiac activity signal associated with a ventricular chamber, wherein the FF cardiac activity signal includes the signal indicative of ventricular activity.

3. The system of claim 2, wherein the electrodes further comprise at least three electrodes (234, 240, 242), and the one or more processors (182) are further configured to select two of the at least three electrodes (234, 240, 242)to maximize the FF cardiac activity signal relative to the atrial events.

4. The system of any one of claims 1 to 3, wherein the LIMD (102) further comprises a heart sound (HS) sensor (154, 188) configured to detect a heart sound signal that comprises an S1 heart sound, wherein the signal indicative of ventricular activity is based on the heart sound signal.

5. The system of any one of claims 1 to 4, wherein the one or more processors (182) are further configured to:
determine the atrial events over a second detection period;
determine the surrogate AV intervals over the second period; and
update the trending of the surrogate AV intervals over time to generate updated diagnostic data.

6. The system of any one of claims 1 to 4, wherein the one or more processors (182) are further configured to transmit the diagnostic data or the surrogate AV intervals to the external device (109).

7. The system of any one of claims 1 to 6, wherein the one or more processors (182) are further configured to:
transition the LIMD (102) from a non-dual chamber operating mode to a dual-chamber operating mode;
receive the signal indicative of ventricular activity from a second LIMD (104); and
restore a previous operational mode of the LIMD (102) at an expiration of the detection period.

8. The system of claim 7, wherein the one or more processors (182) are further configured to:
enable implant-to-implant (i2i) communication between the LIMD (102) and the second LIMD (104); and
in response to restoring the previous operation mode, deactivate the i2i communication.

9. The system of any one of claims 1 to 8, wherein the one or more processors (182) are further configured to generate the alert to prompt at least one of: reprogramming the LIMD (102), implanting a second LIMD (104) in a ventricular chamber, or reprogramming a second LIMD (104) implanted in a ventricular chamber.

10. The system of any one of claims 1 to 9, wherein the evaluate the diagnostic data comprises the one or more processors (182) applying a machine learning model trained to classify progression of AV conduction disease.

11. The system of any one of claims 1 to 10, wherein the one or more processors (182) are further configured to determine that no surrogate AV interval associated with a first atrial event can be determined when the signal indicative of ventricular activity is not detected within a predetermined timeout window following the first atrial event or when a second atrial event is detected before i) the signal indicative of ventricular activity is detected or ii) the predetermined timeout window expires.

12. The system of any one of claims 1 to 11, wherein the atrial events comprise at least one of atrial pace (AP) events or atrial sense (AS) events.

13. The system of any one of claims 1 to 12, wherein the one or more criteria is programmable.

14. The system of claim 1 to 13, wherein the diagnostic data comprises at least one of i) average deviation AV interval, ii) standard deviation AV interval, iii) minimum and maximum AV interval, iv) average of the difference between AV intervals from consecutive cardiac cycles, v) standard deviation of the difference between AV intervals from consecutive cardiac cycles, vi) percentage of cycles without FF CA detection, vii) binned histograms of AV intervals, or viii) binned histograms of differences between AV intervals from consecutive cardiac cycles.

15. The system of claim 1 to 14, wherein the diagnostic data can be managed as moving-window statistics or exponentially filtered statistics.
